# EUROPEAN PATENT APPLICATION

(11) **EP 4 796 179 A2**
(43) Date of publication of application: **26.08.2026**
(21) Application number: 26174600.2
(22) Date of filing: 03.11.2021
(51) Int. Cl.: A61P 31/14

(54) **SOLID FORMULATION**

(30) Priority: 04.11.2020 WO PCT/CN2020/126596
(62) Document number(s) of the earlier application(s) in accordance with Art. 76 EPC: 21809914.1 / 4 240 331
(71) Applicant: Katholieke Universiteit Leuven, 3000 Leuven (BE)
(72) Inventor: ZHU, Donghua, Shanghai, 200233 (CN); KIMPE, Kristof Leonard, 2340 Beerse (BE); ANDERSEN, Sune Klint, 2340 Beerse (BE); RAVELINGIEN, Matthieu Jean M, 2340 Beerse (BE); SOMERS, Ivan Henri M, 2340 Beerse (BE)
(74) Representative: De Clercq & Partners

(57) **Abstract**

The invention relates to pharmaceutical formulations comprising an active pharmaceutical ingredient; and one of or a combination of methacrylic acid copolymer, or a cellulose derivative wherein the active pharmaceutical ingredient is a dengue viral replication inhibitor. Solid dosage forms comprising said pharmaceutical formulations, processes for preparing these and their use in methods of prevention and/or treatment and/or inhibition of viral replication are also described.

## Description

### FIELD OF THE INVENTION

The present invention relates to pharmaceutical formulations comprising: an active pharmaceutical ingredient; and methacrylic acid copolymer, hydroxypropyl methylcellulose or a combination thereof, and solid dosage forms comprising said pharmaceutical formulations. The invention also relates to processes of preparation of such pharmaceutical formulations and to the use of such pharmaceutical formulations for the prevention or the treatment of a disease, syndrome, condition, or disorder.

### BACKGROUND OF THE INVENTION

Flaviviruses, which are transmitted by mosquitoes or ticks, cause life-threatening infections in man, such as encephalitis and hemorrhagic fever. Four distinct, but closely related serotypes of the flavivirus dengue (Dengue virus) are known. WO 2016/180696 discloses active pharmaceutical agents which show high potent activity against all four (4) serotypes of the Dengue virus.

Many active pharmaceutical ingredients (API) have properties such as hydrophobicity and instability leading to challenges in providing suitable pharmaceutical formulations.

There exists a need for improved pharmaceutical formulations of active pharmaceutical ingredients, such as the dengue viral replication inhibitors described in WO 2016/180696.

### SUMMARY OF THE INVENTION

The present invention is directed to a pharmaceutical formulation comprising
a) an active pharmaceutical ingredient (API); and
b1) methacrylic acid copolymer, or
b2) a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC),
wherein the API is a dengue viral replication inhibitor.

Embodiments of the invention include a pharmaceutical formulation as described herein, wherein the API is a dengue viral replication inhibitor.

The invention also provides a solid dosage form comprising a pharmaceutical formulation as described herein.

In embodiments in which the API is a dengue viral replication inhibitor, the invention provides methods for treating or preventing a disease, syndrome, condition, or disorder in a subject, including a mammal and/or human in which the disease, syndrome, condition, or disorder is a dengue viral infection, using pharmaceutical formulations and solid dosage forms described herein.

In certain embodiments, the invention is directed to method for inhibiting dengue viral replication in a mammal and/or human infected with dengue virus or at a risk of being infected with dengue virus.

The present invention is also directed to the use of such pharmaceutical formulations and/or solid dosage in the preparation of a medicament wherein the medicament is prepared for treating or preventing dengue viral infections.

The present invention is also directed to such pharmaceutical formulations and/or solid dosage for use as a medicament. In another embodiment, the present invention is directed to pharmaceutical formulations and/or solid dosage forms described herein for use in the treatment or prevention of dengue viral infections.

In certain embodiments, the present invention is directed to pharmaceutical formulations and/or solid dosage forms described herein for use in the inhibition of dengue viral replication in a mammal and/or human.

The invention also provides a process for preparing a pharmaceutical formulation as described herein, the process comprising the steps of:
a) dissolving the API in a solvent to form a solution;
b) mixing methacrylic acid copolymer or hydroxypropyl methylcellulose or a combination thereof with the solution formed in step a) thereby obtaining a mixture;
c) spray drying the mixture to obtain a solid dispersion;
d) optionally blending the solid dispersion with at least one pharmaceutically acceptable excipient;
to provide a pharmaceutical formulation as described herein.

The invention also provides a process for preparing a solid dosage form described herein, the process comprising the steps of:
a) dissolving the API in a solvent to form a solution;
b) mixing methacrylic acid copolymer or hydroxypropyl methylcellulose or a combination thereof with the solution formed in step a) thereby obtaining a mixture;
c) spray drying the mixture to obtain a solid dispersion;
d) optionally blending the solid dispersion with at least one pharmaceutically acceptable excipient;
e) compressing the blend into a tablet;
to provide a solid dosage form as described herein.

In certain embodiments, the invention provides a process for preparing a pharmaceutical formulation or a solid dosage form as described herein, the process comprising combining the active pharmaceutical ingredient with a methacrylic acid copolymer or with a cellulose derivative (e.g., hydroxypropyl methylcellulose) to form the pharmaceutical formulation or the solid dosage form.

### BRIEF DESCRIPTION OF THE DRAWINGS

The summary, as well as the following detailed description, is further understood when read in conjunction with the appended drawings. For the purpose of illustrating the invention, there are shown in the drawings exemplary embodiments of the invention; however, the invention is not limited to the specific disclosure of the drawings.
**Figure 1** is a plot of % of API dissolved against incubation time for solid dispersions of concepts 1-7, prepared in Example 1. The plot shows a 2-phase dissolution profile in Simulated Gastric Fluid (SGF) and Fasted State Simulated Intestinal Fluid (FaSSIF).
**Figure 2** is a plot of % of API dissolved against incubation time for solid dispersions of concepts 8-2, prepared in Example 1. The plot shows a 2-phase dissolution profile in Simulated Gastric Fluid (SGF) and Fasted State Simulated Intestinal Fluid (FaSSIF).
**Figure 3A and 3B** are plots of % of API dissolved against incubation time (Figure 3A) and weight (in mg) of API dissolved against incubation time (Figure 3B) for solid dispersions prepared in Example 5B. The plots show a 2-phase dissolution profile in Simulated Gastric Fluid (SGF -left of the vertical dotted line) and Fasted State Simulated Intestinal Fluid (FaSSIF -right of the vertical dotted line).
**Figure 4** is a plot of % of API dissolved against incubation time for solid dispersions prepared in Example 5B. The plot shows a 2-phase dissolution profile in Simulated Gastric Fluid (SGF - left of the vertical dotted line) and Fed State Simulated Intestinal Fluid (FeSSIF - right of the vertical dotted line).

### DETAILED DESCRIPTION OF THE INVENTION

The disclosure may be more fully appreciated by reference to the following description, including the following glossary of terms and the concluding examples. It is to be appreciated that certain features of the disclosed pharmaceutical formulations and methods which are, for clarity, described herein in the context of separate aspects, may also be provided in combination in a single aspect. Conversely, various features of the disclosed pharmaceutical formulations and methods that are, for brevity, described in the context of a single aspect, may also be provided separately or in any sub-combination.

Some of the quantitative expressions given herein are not qualified with the term "about. " It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value. The terms "about" or "approximately" as used herein, when referring to a numerical value or range, allow for a degree of variability in the value or range, for example, within 10% (i.e., ±10%), within 5% (i.e., ±5%), or within 2.5% (i.e., ± 2.5%) of a stated value or of a stated limit of a range.

Throughout the description and claims of this specification, the words "comprise" and "contain" and variations of the words, for example "comprising" and "comprises", mean "including but not limited to", and are not intended to (and do not) exclude other components.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

All references cited in the present specification are hereby incorporated by reference in their entirety. In particular, the teachings of all references herein specifically referred to are incorporated by reference.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art.

For use in medicine, cocrystals or salts of the API such as salts of compounds of Formula (I) as disclosed herein refer to non-toxic "pharmaceutically acceptable salts." "Pharmaceutically acceptable" may mean approved or approvable by a regulatory agency of the Federal or a state government or the corresponding agency in countries other than the United States, or that is listed in the U. S. Pharmacopoeia or other generally recognized pharmacopoeia for use in animals, and more particularly, in humans.

Other salts may, however, be useful in the preparation of the API such as compounds of Formula (I) or of their pharmaceutically acceptable salt forms thereof. Suitable pharmaceutically acceptable salts of the API such as compounds of Formula (I) include acid addition salts that can, for example, be formed by mixing a solution of the compound with a solution of a pharmaceutically acceptable acid such as, hydrochloric acid, sulfuric acid, fumaric acid, maleic acid, succinic acid, acetic acid, benzoic acid, citric acid, tartaric acid, carbonic acid or phosphoric acid. Furthermore, where the API such as compounds of Formula (I) carry an acidic moiety, suitable pharmaceutically acceptable salts thereof may include alkali metal salts such as, sodium or potassium salts; alkaline earth metal salts such as, calcium or magnesium salts; and salts formed with suitable organic ligands such as, quaternary ammonium salts. Thus, representative pharmaceutically acceptable salts include acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide, and valerate.

Representative acids and bases that may be used in the preparation of pharmaceutically acceptable salts include acids including acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1 S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and bases including ammonia, L-arginine, benethamine, benzathine, calcium hydroxide, choline, deanol, diethanolamine, diethylamine, 2-(diethylamino)-ethanol, ethanolamine, ethylenediamine, *N*-methyl-glucamine, hydrabamine, *1H*-imidazole, L-lysine, magnesium hydroxide, 4-(2-hydroxyethyl)-morpholine, piperazine, potassium hydroxide, 1-(2-hydroxyethyl)-pyrrolidine, sodium hydroxide, triethanolamine, tromethamine, and zinc hydroxide.

Where the API such as compounds of Formula (I) have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the compounds may exist as polymorphs and as such are intended to be included in the present invention. In addition, some of the compounds may form solvates with water (i.e., hydrates) or organic solvents, and such solvates are also intended to be encompassed within the scope of this invention. The skilled artisan will understand that the term compound as used herein, is meant to include solvated compounds of Formula (I).

Where the processes for the preparation of the API such as compounds of Formula (I) give rise to mixture of stereoisomers, these isomers may be separated by conventional techniques such as, preparative chromatography. The compounds may be prepared in racemic form, or individual enantiomers may be prepared either by enantiospecific synthesis or by resolution. The compounds may, for example, be resolved into their component enantiomers by standard techniques such as, the formation of diastereomeric pairs by salt formation with an optically active acid such as, (-)-di-p-toluoyl-d-tartaric acid and/or (+)-di-p-toluoyl-l-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral stationary phase or iso chiral column.

In one embodiment of the pharmaceutical formulation of the present invention, the API is a compound of Formula (I), wherein said compound is a compound comprising, consisting of, and/or consisting essentially of the (+)-enantiomer wherein said compound is substantially free from the (-)-isomer. In the present context, substantially free means less than about 25 wt%, preferably less than about 10 wt%, more preferably less than about 5 wt%, even more preferably less than about 2 wt% and even more preferably less than about 1 wt% of the (-)-isomer calculated as $% \left(+\right) - enantiomer = \frac{\left(mass\left(+\right)-enantiomer\right)}{\left(mass\left(+\right)-enantiomer\right) + \left(mass\left(-\right)-enantiomer\right)} \times 100 .$

In another embodiment of the pharmaceutical formulation of the present invention, the API is a compound of Formula (I), wherein said compound is a compound comprising, consisting of, and consisting essentially of the (-)-enantiomer wherein said compound is substantially free from the (+)-isomer. In the present context, substantially free from means less than about 25 wt%, preferably less than about 10 wt%, more preferably less than about 5 wt%, even more preferably less than about 2 wt% and even more preferably less than about 1 wt% of the (+)-isomer calculated as $% \left(-\right) - enantiomer = \frac{\left(mass\left(-\right)-enantiomer\right)}{\left(mass\left(+\right)-enantiomer\right) + \left(mass\left(-\right)-enantiomer\right)} \times 100 .$

During any of the processes for preparation of the compounds of the various embodiments of the present invention, it may be necessary and/or desirable to protect sensitive or reactive groups on any of the molecules concerned. This may be achieved by means of conventional protecting groups such as those described in Protective Groups in Organic Chemistry, Second Edition, J.F.W. McOmie, Plenum Press, 1973; T.W. Greene & P.G.M. Wuts, Protective Groups in Organic Synthesis, John Wiley & Sons, 1991; and T.W. Greene & P.G.M. Wuts, Protective Groups in Organic Synthesis, Third Edition, John Wiley & Sons, 1999. The protecting groups may be removed at a convenient subsequent stage using methods known from the art.

Unless stated otherwise, the terms "wt%" and "weight percent" are used interchangeably to refer to the concentration of an ingredient (e.g., excipient or active pharmaceutical ingredient) by weight of the pharmaceutical formulation.

The term "room temperature" (RT) refers to a temperature of from about 15 °C to about 30°C, in particular from about 20 °C to about 30 °C. Preferably, room temperature is a temperature of about 25 °C.

An average molecular weight may, for example, refer to a number average or weight average molecular weight. Average molecular weight may, for example, be measured using gel permeation chromatography or mass spectrometry.

The term "subject" refers to an animal, preferably a mammal, most preferably a human, who has been the object of treatment, observation or experiment.

The terms "active compound", "active ingredient" and "active pharmaceutical ingredient" are herein used interchangeably.

The term "therapeutically effective amount" refers to an amount of an active compound or pharmaceutical agent which elicits the biological or medicinal response in a tissue system, animal or human that is being sought by a researcher, veterinarian, medical doctor or other clinician, including reduction or inhibition of an enzyme or a protein activity, or ameliorating symptoms, alleviating conditions, slowing or delaying disease progression, or preventing a disease.

In embodiments in which the API is a dengue viral replication inhibitor, the term "therapeutically effective amount" may refer to the amount of a formulation of the present invention that, when administered to a subject, is effective to at least partially alleviate, inhibit, prevent, and/or ameliorate a condition, or a disorder or a disease caused by a Dengue virus in said subject.

As used herein, the term "Dengue virus" refers to the single positive-stranded RNA virus of the family Flaviviridae; four distinct, but closely related serotypes of the flavivirus dengue are known, so-called DENV-1, -2, -3, and -4. Flaviviruses, which are transmitted by mosquitoes or ticks, cause life-threatening infections in man, such as encephalitis and hemorrhagic fever.

As used herein, the term "dengue viral replication inhibitor" refers to an agent that inhibits or reduces at least one condition, symptom, disorder, and/or disease caused by a Dengue virus.

As used herein, unless otherwise noted, the term "affect" or "affected" (when referring to a disease, syndrome, condition or disorder that is affected by the inhibition of a Dengue virus replication) includes a reduction in the frequency and/or severity of one or more symptoms or manifestations of said disease, syndrome, condition or disorder; and/or includes the prevention of the development of one or more symptoms or manifestations of said disease, syndrome, condition or disorder or the development of the disease, condition, syndrome or disorder.

As used herein, the term "treat", "treating", or "treatment" of any disease, condition, syndrome or disorder refers, in one embodiment, to ameliorating the disease, condition, syndrome or disorder (i.e. slowing or arresting or reducing the development of the disease or at least one of the clinical symptoms thereof). In another embodiment, "treat", "treating", or "treatment" refers to alleviating or ameliorating at least one physical parameter including those which may not be discernible by the patient. In a further embodiment, "treat", "treating", or "treatment" refers to modulating the disease, condition, syndrome or disorder either physically (e.g. stabilization of a discernible symptom), physiologically, (e.g. stabilization of a physical parameter), or both. In yet another embodiment, "treat", "treating", or "treatment" refers to preventing or delaying the onset or development or progression of the disease, condition, syndrome or disorder.

As used herein, the terms "prevent", "prevention" and "preventing" refer to the reduction in the risk of acquiring or developing a given condition, disease or disease symptoms, or the reduction or inhibition of the recurrence or said condition, disease or disease symptoms in a subject who is not ill, but who is at risk of being ill. For instance, said subject is or has been to a high risk area or has been or may be near a person with the disease.

As used herein, an "excipient" is an inactive ingredient in a pharmaceutical formulation. Examples of excipients include diluents, wetting agents (e.g., surfactants), binders, glidants, lubricants, disintegrants, and the like.

As used herein, a "disintegrant agent" or "disintegrant" is an excipient that hydrates a pharmaceutical formulation and aids in tablet dispersion. Examples of disintegrant agents include croscarmellose sodium, crospovidone (i.e., cross-linked polyvinyl N-pyrrolidone), sodium starch glycolate, or any combination thereof.

As used herein, a "diluent" or "filler" is an excipient that adds bulkiness to a pharmaceutical formulation. Examples of diluents include lactose, sorbitol, celluloses, calcium phosphates, starches, sugars (e.g., mannitol, sucrose, or the like) or any combination thereof.

As used herein, a "wetting agent" or a "surfactant" is an excipient that imparts pharmaceutical formulations with enhanced solubility and/or wettability. Examples of wetting agents include sodium lauryl sulfate (SLS), sodium stearyl fumarate (SSF), polyoxyethylene 20 sorbitan mono-oleate (i.e. polysorbate 20) (e.g., Tween^{™} or Tween 20), Soluplus^{®}, or any combination thereof.

As used herein, a "binder" is an excipient that imparts a pharmaceutical formulation with enhanced cohesion or tensile strength (e.g., hardness). Examples of binders include dibasic calcium phosphate, sucrose, corn (maize) starch, microcrystalline cellulose, and modified cellulose (e.g., hydroxymethyl cellulose).

As used herein, a "glidant" is an excipient that imparts pharmaceutical formulation with enhanced flow properties. Examples of glidants include colloidal silica and/or talc.

As used herein, a "colorant" is an excipient that imparts a pharmaceutical formulation with a desired color. Examples of colorants include commercially available pigments such as FD&C Blue #1 Aluminum Lake, FD&C Blue #2, other FD&C Blue colors, titanium dioxide, iron oxide, and/or combinations thereof. Other colorants include commercially available pigments such as FD&C Green #3.

As used herein, a "lubricant" is an excipient that is added to pharmaceutical formulation that are pressed into tablets. The lubricant aids in compaction of granules into tablets and ejection of a tablet of a pharmaceutical formulation from a die press. Examples of lubricants include magnesium stearate, stearic acid (stearin), hydrogenated oil, sodium stearyl fumarate, or any combination thereof.

Preferred statements (features) and embodiments of the pharmaceutical formulations, uses and process of this invention are set herein below. Each statement and embodiment of the invention so defined may be combined with any other statement and/or embodiment unless clearly indicated to the contrary. In particular, any feature indicated as being preferred or advantageous may be combined with any other feature or features or statement indicated as being preferred or advantageous. Hereto, the present invention is in particular captured by any one or any combination of one or more of the below numbered aspects and embodiments, with any other statement and/or embodiment.
1. A pharmaceutical formulation, comprising:
   a) an active pharmaceutical ingredient (API); and
   b1) methacrylic acid copolymer, or
   b2) a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC), or a combination thereof. Preferably the cellulose derivative is HPMC.
   wherein the API is a dengue viral replication inhibitor.
2. The pharmaceutical formulation of statement 1, wherein the pharmaceutical formulation is a solid formulation.
3. The pharmaceutical formulation of any one of statements 1 or 2, wherein the API and the methacrylic acid copolymer are present in said formulation in a ratio of from 4:1 w/w to 1:9 w/w, preferably from 3.9:1 w/w to 1:8 w/w; preferably from 3.8:1 w/w to 1:7 w/w; from 3.7:1 w/w to 1:6 w/w; preferably from 3:6:1 w/w to 1:5 w/w, preferably from 3.5:1 w/w to 1:4.5 w/w; preferably from 3:1 w/w to 1:4 w/w; preferably from 2.5:1 w/w to 1:3.5 w/w; preferably from 2:1 w/w to 1:3 w/w; preferably from 2.5:1 w/w to 1:2.5 w/w, preferably from 2:1 w/w to 1:2 w/w; preferably from 1.5:1 w/w to 1:1.5 w/w or a ratio comprised within any two ratios mentioned herein, or a ratio range or sub-range within any two ratios mentioned herein.
4. The pharmaceutical formulation of any one of statements 1 or 2, wherein the API and the cellulose derivative (e.g., hydroxypropyl methylcellulose) are present in said formulation in a ratio of from 4:1 w/w to 1:5 w/w, preferably from 3.5:1 w/w to 1:4.5 w/w; preferably from 3:1 w/w to 1:4 w/w; preferably from 2.5:1 w/w to 1:3.5 w/w; preferably from 2:1 w/w to 1:3 w/w; preferably from 2.5:1 w/w to 1:2.5 w/w, preferably from 2:1 w/w to 1:2 w/w; preferably from 1.5:1 w/w to 1:1.5 w/w or a ratio comprised within any two ratios mentioned herein, or a ratio range or sub-range within any two ratios mentioned herein.
5. The pharmaceutical formulation of any one of statements 1 to 4, wherein the methacrylic acid copolymer is a copolymer of acrylic-and/or methacrylic acids/ester such as those compounds sold under the trade name Eudragit^{®} including but not limited to Eudragit^{®} L-100, Eudragit^{®} E-100, Eudragit^{®} L-100-55 or any mixtures thereof.
6. The pharmaceutical formulation of any one of statements 1 to 5, wherein the methacrylic acid copolymer is selected from the group comprising a copolymer of methacrylic acid and methyl methacrylate; a copolymer of methacrylic acid and ethyl acrylate; and any mixtures thereof.
7. The pharmaceutical formulation of any one of statements 1 to 6, wherein the methacrylic acid copolymer is a copolymer of methacrylic acid and methyl methacrylate.
8. The pharmaceutical formulation to statement 7, wherein the molar ratio of methacrylic acid to methyl methacrylate in the copolymer is 0.5:2 to 0.6 :1.5 , preferably 0.8:1.3 to 1.2:1, more preferably around 1:1.
9. The pharmaceutical formulation of any one of statements 1 to 8, wherein the methacrylic acid copolymer is poly(methacrylic acid-co-methyl methacrylate) 1:1 (EUDRAGIT^{®} L100).
10. The pharmaceutical formulation of any one of statements 1 to 9, wherein cellulose derivative is hydroxypropyl methylcellulose having a viscosity ranging between 3 and 5000 mPa.s in 2 wt% solution in H₂O at 25°C, such as HPMC E5, HPMC E6, HPMC E15, HPMC E50, HPMC K3, HPMC K4M, HPMC-AS and mixtures thereof.
11. The pharmaceutical formulation of any one of statements 1 to 10, wherein the cellulose derivative is hydroxypropyl methylcellulose having a viscosity ranging between 3 and 500 mPa.s in 2 wt% solution in H₂O at 25°C such as HPMC E5, HPMC E6, HPMC E15, HPMC E50, and mixtures thereof.
12. The pharmaceutical formulation of any one of statements 1 to 11, wherein the cellulose derivative has a viscosity ranging between 3 and 50 mPa.s in 2 wt% solution in H₂O at 25°C. Preferably said cellulose derivative is hydroxypropyl methylcellulose selected from HPMC E5, HPMC E6, HPMC E15, HPMC E50, and mixtures thereof.
13. The pharmaceutical formulation of any one of statements 1 to 12, wherein the formulation comprises at most 50 wt%, preferably at most 40 wt%, more preferably at most 30 wt%, most preferably at most 25 wt%, even most preferably 20 wt% of the API relative to the total weight of the formulation.
14. The pharmaceutical formulation of any one of statements 1 to 13, wherein the formulation comprises from 0.1 wt% to 50 wt%, preferably from 1 wt% to 40 wt%, more preferably from 2.5 wt% to 30 wt%, most preferably from 5 wt% to 25 wt% of the API relative to the total weight of the formulation.
15. The pharmaceutical formulation of any one of statements 1 to 14, wherein the formulation further comprises one or more pharmaceutically acceptable excipients selected from disintegrants, binders, diluents, lubricants, stabilizers, wetting agents, glidants, osmotic agents, colorants, plasticizers, and coatings.
16. The pharmaceutical formulation of any one of statements 1 to 15, wherein the formulation further comprises one or more excipients; wherein the formulation comprises at most 80 wt%, preferably at most 70 wt%, preferably at most 60 wt% of the one or more excipients relative to the total weight of the formulation; and/or wherein the formulation comprises at least 10 wt%, preferably at least 20 wt%, preferably at least 30 wt% of the one or more excipients relative to the total weight of the formulation.
17. The pharmaceutical formulation of any one of statements 1 to 16, wherein the formulation further comprises one or more diluents; wherein the formulation comprises at most 95 wt%, preferably at most 80 wt%, preferably at most 75 wt% of the diluent relative to the total weight of the formulation; and/or wherein the formulation comprises at least 5 wt%, preferably at least 10 wt%, preferably at least 15 wt% of the diluent relative to the total weight of the formulation.
18. The pharmaceutical formulation of any one of statements 1 to 17, wherein the formulation further comprises one or more disintegrants; wherein the formulation comprises at most 30 wt%, preferably at most 20 wt%, preferably at most 15 wt%, preferably at most 10 wt% of the disintegrant relative to the total weight of the formulation; and/or wherein the formulation comprises at least 1 wt%, preferably at least 2.5 wt%, preferably at least 5 wt%, preferably least 8 wt% of the disintegrant relative to the total weight of the formulation.
19. The pharmaceutical formulation of any one of statements 1 to 18, wherein the formulation further comprises one or more binders; wherein the formulation comprises at most 50 wt%, preferably at most 45 wt%, preferably at most 40 wt%, more preferably at most 35 wt% of the binder relative to the total weight of the formulation; and/or wherein the formulation comprises at least 5 wt%, preferably at least 10 wt%, preferably at least 15 wt%, more preferably at least 20 wt%, most preferably 25 wt% of the binder relative to the total weight of the formulation.
20. The pharmaceutical formulation of any one of statements 1 to 19, wherein the formulation further comprises one or more lubricants; wherein the formulation comprises at most 5.5 wt%, preferably at most 3.5 wt%, preferably at most 2 wt% of the lubricant relative to the total weight of the formulation; and/or wherein the formulation comprises at least 0.5 wt%, preferably at least 1 wt%, preferably at least 1.5 wt% of the lubricant relative to the total weight of the formulation.
21. The pharmaceutical formulation of any one of statements 1 to 20, wherein the formulation further comprises one or more wetting agent; wherein the formulation comprises at most 5.5 wt%, preferably at most 3.5 wt%, preferably at most 2 wt% of the wetting agent relative to the total weight of the formulation; and/or wherein the formulation comprises at least 0.5 wt%, preferably at least 1 wt%, preferably at least 1.5 wt% of the wetting agent relative to the total weight of the formulation.
22. The pharmaceutical formulation of any one of statements 1 to 21, wherein the formulation further comprises one or more glidant; wherein the formulation comprises at most 10 wt%, preferably at most 8 wt%, preferably at most 5 wt% of the glidant relative to the total weight of the formulation; and/or wherein the formulation comprises at least 0.1 wt%, preferably at least 1 wt%, preferably at least 2 wt% of the glidant relative to the total weight of the formulation.
23. The pharmaceutical formulation of any one of statements 1 to 22, wherein the formulation comprises a plurality of granules forming an intragranular phase of the formulation and one or more excipients forming an extragranular phase of the formulation.
24. The pharmaceutical formulation of statement 23, wherein the formulation comprises at least 15 wt%; at least 20 wt%; at least 28 wt%; at least 34 wt% of intragranular phase by weight of the pharmaceutical formulation.
25. The pharmaceutical formulation of any one of statements 23 or 24, wherein the formulation comprises at most 99 wt%, at most 93 wt%, at most 85 wt%, at most 80 wt%, at most 74 wt%; at most 73 wt%; at most 67 wt%; at most 63 wt%; at most 60 wt% at most 53 wt% of intragranular phase by weight of the pharmaceutical formulation.
26. The pharmaceutical formulation of any one of statements 23 to 25, wherein the formulation comprises an intragranular phase, and wherein the intragranular phase comprising from 1 wt% to 70 wt%; 2 wt% to 60 wt%; 3 wt% to 55 wt%; 4 wt% to 50 wt%; 5 wt% to 45 wt%,; preferably from 5 wt% to 40 wt%; preferably from 10 wt% to 35 wt%; preferably from 15 wt% to 30 wt% of API by the total weight of the pharmaceutical formulation.
27. The pharmaceutical formulation of any one of statements 23 to 26, wherein the formulation comprises an intragranular phase, and wherein the intragranular phase comprises from 5 wt% to 60 wt%; preferably from 8 wt% to 50 wt%; preferably from 15 wt% to 45 wt% of methacrylic acid copolymer or hydroxypropyl methylcellulose or a mixture thereof by the total weight of the pharmaceutical formulation.
28. The pharmaceutical formulation of any one of statements 23 to 27, wherein the formulation comprises an intragranular phase, and wherein the intragranular phase comprises from 5 wt% to 60 wt%; preferably from 10 wt% to 60 wt%; preferably from 10 wt% to 50 wt%; preferably from 15 wt% to 50 wt% of filler by the total weight of the pharmaceutical formulation.
29. The pharmaceutical formulation of any one of statements 23 to 28, wherein the formulation comprises an intragranular phase comprising from 1 wt% to 10 wt%; preferably from 1 wt% to 9 wt%, preferably from 1.7 wt% to 8 wt% of disintegrant by the total weight of the pharmaceutical formulation.
30. The pharmaceutical formulation of any one of statements 23 to 29, wherein the formulation comprises an intragranular phase comprising from 0.1 wt% to 5 wt%; preferably from 0.2 wt% to 4.7 wt%; preferably from 0.5 to 4.5 wt% of glidant by the total weight of the pharmaceutical formulation.
31. The pharmaceutical formulation of any one of statements 23 to 30, wherein the formulation comprises an intragranular phase comprising from 0.1 wt% to 5 wt%; preferably from 0.2 wt% to 4.7 wt%; preferably from 0.5 to 4.5 wt% of surfactant by the total weight of the pharmaceutical formulation.
32. The pharmaceutical formulation of any one of statements 23 to 31, wherein the formulation comprises an intragranular phase comprising from 0.1 wt% to 3 wt%; preferably from 0.2 wt% to 2.7 wt%; preferably from 0.5 to 2.5 wt% of lubricant by the total weight of the pharmaceutical formulation.
33. The pharmaceutical formulation of any one of statements 23 to 32, wherein the formulation comprises an intragranular phase, the intragranular phase comprising: (i) from 5 wt% to 45 wt%,; preferably from 5 wt% to 40 wt%; preferably from 10 wt% to 35 wt%; preferably from 15 wt% to 30 wt% of API by the total weight of the pharmaceutical formulation; (ii) from 5 wt% to 60 wt%; preferably from 8 wt% to 50 wt%; preferably from 15 wt% to 45 wt% of methacrylic acid copolymer or hydroxypropyl methylcellulose or a mixture thereof by the total weight of the pharmaceutical formulation; (iii) from 5 wt% to 60 wt%; preferably from 10 wt% to 60 wt%,; preferably from 10 wt% to 50 wt%; preferably from 15 wt% to 50 wt% of filler by the total weight of the pharmaceutical formulation; (iv) from 1 wt% to 10 wt%,; preferably from 1 wt% to 9 wt%, preferably from 1.7 wt% to 8 wt% of disintegrant by the total weight of the pharmaceutical formulation; (v) from 0.1 wt% to 5 wt%; preferably from 0.2 wt% to 4.7 wt%; preferably from 0.5 to 4.5 wt% of glidant by the total weight of the pharmaceutical formulation; (vi) from 0.1 wt% to 5 wt%; preferably from 0.2 wt% to 4.7 wt%; preferably from 0.5 to 4.5 wt% of surfactant by the total weight of the pharmaceutical formulation; and (vii) from 0.1 wt% to 3 wt%; preferably from 0.2 wt% to 2.7 wt%; preferably from 0.5 to 2.5 wt% of lubricant by the total weight of the pharmaceutical formulation.
34. The pharmaceutical formulation of any one of statements 23 to 33, wherein the pharmaceutical formulation comprises at least 5wt%; at least 7 wt%; at least 10 wt%; at least 15 wt%; at least 20 wt%; at least 28 wt%; at least 34 wt% of extragranular phase by the total weight of the pharmaceutical formulation.
35. The pharmaceutical formulation of any one of statements 23 to 34, wherein the pharmaceutical formulation comprises at most 74 wt%; at most 73 wt%; at most 67 wt%; at most 63 wt%; at most 53 wt%, at most 40 wt% of extragranular phase by the total weight of the pharmaceutical formulation.
36. The pharmaceutical formulation of any one of statements 23 to 35, wherein the formulation comprises an extragranular phase, the extragranular phase comprising from 0.1 wt% to 5.0 wt%; preferably from 0.2 wt% to 4.7 wt%; preferably from 0.5 wt% to 3.5 wt% of disintegrant by the total weight of the pharmaceutical formulation.
37. The pharmaceutical formulation of any one of statements 23 to 36, wherein the formulation comprises an extragranular phase, the extragranular phase comprising from 0.1 wt% to 3 wt%; preferably from 0.2 wt% to 2.7 wt%; preferably from 0.5 wt% to 2.5 wt% of lubricant by the total weight of the pharmaceutical formulation.
38. The pharmaceutical formulation of any one of statements 23 to 37, wherein the formulation comprises an extragranular phase, the extragranular phase comprising from 0.1 wt% to 55 wt%; from 0.2 wt% to 50 wt%; from 0.3 wt% to 45 wt%; from 0.4 wt% to 40 wt%; from 0.5 wt% to 35 wt%; from 0.6 wt% to 30 wt%; from 0.7 to 25 wt%; from 0.8 wt% to 20 wt%; 1 wt% to 15 wt%; from 1.3 wt% to 12 wt%; from 2 to 10; from 2.5 wt% to 9 wt%; from 3 wt% to 8 wt%; from 4 wt% to 7 wt%; from 5 wt% to 6 wt% of filler by the total weight of the pharmaceutical formulation.
39. The pharmaceutical formulation of any one of statements 23 to 38, wherein the formulation comprises an extragranular phase, the extragranular phase comprising: (a) from 0.1 wt% to 5.0 wt%; preferably from 0.2 wt% to 4.7 wt%; preferably from 0.5 wt% to 3.5 wt%; more preferably from 1 wt% to 3 wt% of disintegrant by the total weight of the pharmaceutical formulation; (b) from 0.1 wt% to 3 wt%; preferably from 0.2 wt% to 2.7 wt%; preferably from 0.5 wt% to 2.5 wt% of lubricant by the total weight of the pharmaceutical formulation; and (c) from 1 wt% to 15 wt%; preferably from 1.3 wt% to 12 wt%; preferably from 2.5 wt% to 8 wt%; more preferably from 3 to 5 wt% of filler by the total weight of the pharmaceutical formulation.
40. The pharmaceutical formulation of any one of statements 1 to 39, wherein the API is a compound of Formula (I) 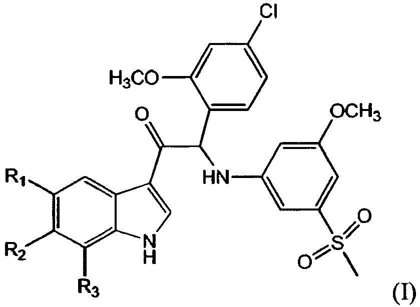 a stereo-isomeric form, a pharmaceutically acceptable salt, solvate or polymorph thereof; said compound is selected from the group wherein:
   R₁ is H, R₂ is F and R₃ is H or CH₃,
   R₁ is H, CH₃ or F, R₂ is OCH₃ and R₃ is H and
   R₁ is H, R₂ is OCH₃ and R₃ is CH₃,
   R₁ is CH₃, R₂ is F and R₃ is H,
   R₁ is CF₃ or OCF₃, R₂ is H and R₃ is H,
   R₁ is OCF₃, R₂ is OCH₃ and R₃ is H and
   R₁ is OCF₃, R₂ is H and R₃ is CH₃.
41. The pharmaceutical formulation according to statement 40, wherein the compound of Formula (I) is: 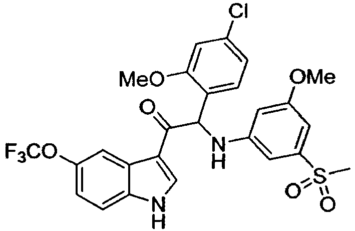 or a stereo-isomeric form, a pharmaceutically acceptable salt, solvate or polymorph thereof.
42. A solid dosage form comprising the pharmaceutical formulation of any one of statements 1 to 41.
43. The solid dosage form of statement 42, wherein the dosage form is an oral dosage form.
44. The solid dosage form of statements 42 or 43, wherein the dosage form is a tablet.
45. The solid dosage form of any one of statements 42-44, wherein the formulation comprises from 0.5 to 1000 mg of the API; preferably from 1 to 900 mg of the API; preferably from 2 to 800 mg of the API; preferably from 3 to 700 mg of the API; preferably from 4 to 600 mg of the API; preferably from 5 to 500 mg of the API; preferably from 6 to 400 mg of the API; preferably from 7 to 300 mg of the API; preferably from 8 to 200 mg of the API; preferably from 9 to 100 mg of the API; preferably from 10 to 50 mg of the API.
46. The solid dosage form of any one of statements 42 to 45, wherein the formulation comprises at least 0.1 mg, at least 5 mg, at least 10 mg, at least 20 mg, at least 50 mg, at least 100 mg, at least 200 mg, at least 300 mg, at least 400 mg, at least 500 mg or at least 1000 mg of the API; preferably the API is: 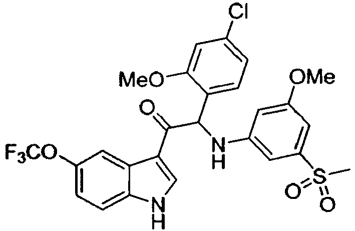 or a stereo-isomeric form, a pharmaceutically acceptable salt, solvate or polymorph thereof.
47. A method of treating or preventing of dengue viral infections, comprising administering to a subject in need thereof a therapeutically effective amount of a pharmaceutical formulation of any one of statements 1 to 41, or administering to a subject in need thereof a therapeutically effective amount of the solid dosage form of any one of statements 42 to 46.
48. The use of a pharmaceutical formulation of any one of statements 1 to 41 for the preparation of a medicament for treating or preventing of dengue viral infections.
49. The use of a solid dosage form of any one of statements 42 to 46 for the preparation of a medicament for treating or preventing of dengue viral infections.
50. A pharmaceutical formulation of any one of statements 1 to 41 for use as a medicament.
51. A solid dosage form of any one of statements 42 to 46 for use as a medicament.
52. A pharmaceutical formulation of any one of statements 1 to 41 for use in a method for treating or preventing of dengue viral infections.
53. A solid dosage form of any one of statements 42 to 46 for use in a method for treating or preventing of dengue viral infections.
54. A process for preparing a pharmaceutical formulation according to any one of statements 1 to 41, comprising the steps of:
   a) dissolving the API in a solvent to form a solution;
   b) mixing the methacrylic acid copolymer or hydroxypropyl methylcellulose or a combination thereof with the solution formed in step a) thereby obtaining a mixture;
   c) spray drying the mixture to obtain a solid dispersion;
   d) optionally blending the solid dispersion with at least one pharmaceutically acceptable excipient;
   to provide a pharmaceutical formulation according to any one of statements 1 to 41.
55. The process of any one of statement 54, wherein the API is a compound of Formula (I) 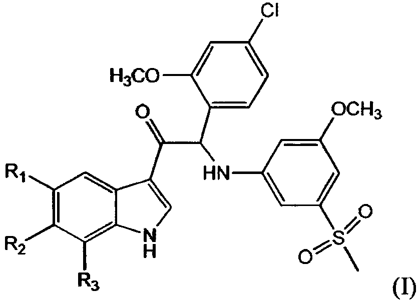 a stereo-isomeric form, a pharmaceutically acceptable salt, solvate or polymorph thereof, said compound is selected from the group wherein:
   R₁ is H, R₂ is F and R₃ is H or CH₃,
   R₁ is H, CH₃ or F, R₂ is OCH₃ and R₃ is H and
   R₁ is H, R₂ is OCH₃ and R₃ is CH₃,
   R₁ is CH₃, R₂ is F and R₃ is H,
   R₁ is CF₃ or OCF₃, R₂ is H and R₃ is H,
   R₁ is OCF₃, R₂ is OCH₃ and R₃ is H and
   R₁ is OCF₃, R₂ is H and R₃ is CH₃.
56. The process of any one of statements 54-55, wherein the API is: 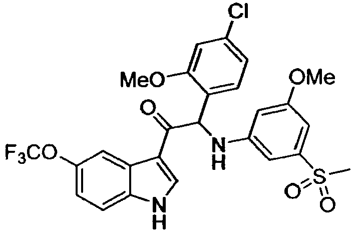 or a stereo-isomeric form, a pharmaceutically acceptable salt, solvate or polymorph thereof.
57. A process for preparing a pharmaceutical formulation according to any one of statements 1 to 41 or a solid dosage form according to any one of statements 42-46, the process comprising combining the active pharmaceutical ingredient with a methacrylic acid copolymer or with a cellulose derivative (e.g., hydroxypropyl methylcellulose) to form the pharmaceutical formulation or the solid dosage form.

### Pharmaceutical formulations

The invention provides a pharmaceutical formulation, comprising:
a) an active pharmaceutical ingredient (API) which is a dengue viral replication inhibitor; and
b1) methacrylic acid copolymer, or
b2) a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC), or a combination thereof.

In some embodiments, the cellulose derivative is HPMC.

In some embodiments, the pharmaceutical formulation according to the invention comprises an API which is a dengue viral replication inhibitor and at least one methacrylic acid copolymer.

As used herein the term "methacrylic acid copolymer", preferably refers to a copolymer of acrylic- and/or methacrylic acids/ester such as those compounds sold under the trade name Eudragit^{®}. Eudragit^{®} is commercially available, for example, from Evonik Healthcare & Nutrition GmbH, Essen, Germany.

Different types of methacrylic acid copolymer can be used, and they include poly(methacrylic acid co-methyl methacrylate) 1:1 (such as Eudragit^{®} L-100, Eudragit^{®} L12.5); poly(methacrylic acid co-methyl methacrylate) 1:2 (such as Eudragit^{®} S-100, Eudragit^{®} S12,5, Eudragit^{®} FS30D); poly(methacrylic acid co-ethyl acrylate) 1:1 (such as Eudragit^{®} L30D55, Eudragit^{®} L100-55); Poly(ethyl acrylate-co-methyl methacrylate-co-trimethylammonioethyl methacrylate chloride) 1:2:0.1 (such as Eudragit^{®} RS30D); poly(ethyl acrylate, methyl methacrylate, trimethylammonioethyl methacrylate chloride) 1:2:0.2 (such as Eudragit^{®} RL30D); poly(ethyl acrylate co-methyl methacrylate) 2:1 (such as Eudragit^{®} NM 30D, or Eudragit NE 30D); cationic copolymer based on dimethylaminoethyl methacrylate, butyl methacrylate, and methyl methacrylate with a ratio of 2:1:1 (Eudragit^{®} E-100) and combinations thereof.

In some embodiments, the methacrylic acid copolymer is selected from the group comprising a copolymer of methacrylic acid and methyl methacrylate; a copolymer of methacrylic acid and ethyl acrylate; and mixture thereof.

In some embodiments, the methacrylic acid copolymer is a copolymer of methacrylic acid and methyl methacrylate. Preferably, the molar ratio of methacrylic acid to methyl methacrylate in the copolymer is 0.5:2 to 2:0.5, preferably 0.8:1 to 1.2:1, (e.g. , 1:1).

In some embodiments, the methacrylic acid copolymer is poly(methacrylic acid-co-methyl methacrylate) 1:1 (EUDRAGIT^{®} L100, CAS number: 25086-15-1).

In some embodiments, the pharmaceutical formulation according to the invention comprises an API which is a dengue viral replication inhibitor and hydroxypropyl methylcellulose.

In some embodiments, the pharmaceutical formulation according to the invention comprises a crystallisation rate inhibitor. The term "crystallisation rate inhibitor" refers to an excipient, for example a polymeric excipient, that is added to the formulation with the aim of inhibiting crystallisation of an API when the formulation is administered to a subject. A crystallisation rate inhibitor may be used to improve the bioavailability of an API where the bioavailability of the crystalline form is significantly lower in comparison to the amorphous/dissolved state. The crystallisation rate inhibitor may be referred to as a crystallisation inhibitor or a stabilizer.

In an embodiment, the crystallisation rate inhibitor is selected from polyvinylpyrrolidone (PVP), a polyvinylpyrrolidone-vinyl acetate copolymer (PVPVA), a poly(meth)acrylate polymer (e.g. methacrylic acid-methyl methacrylate copolymer), a cyclodextrin or a cyclodextrin derivative (e.g. (2-hydroxypropyl)-β-cyclodextrin (HPBCD)), hydroxypropylcellulose, hydroxyethylcellulose, methylcellulose, hydroxypropyl methylcellulose (HPMC), hydroxypropyl methylcellulose acetate succinate (HPMCAS), a polyethylene glycol-polyvinyl acetate-polyvinyl caprolactame graft copolymer, poly(vinyl alcohol), a poloxamer (e.g. poloxamer 188, 338, or 407), and any combinations thereof.

In an embodiment, the crystallisation rate inhibitor is selected from hydroxypropyl methylcellulose (HPMC), hydroxypropyl methylcellulose acetate succinate (HPMCAS), a polyethylene glycol-polyvinyl acetate-polyvinyl caprolactame graft copolymer, polyvinylpyrrolidone (PVP) and a polyvinylpyrrolidone-vinyl acetate copolymer (PVPVA), and a combination thereof. In a further embodiment, the crystallisation rate inhibitor is selected from hydroxypropyl methylcellulose (HPMC) and polyvinylpyrrolidone-vinyl acetate copolymer (PVPVA). The PVPV A may be a copolymer of 1-vinyl-2-pyrrolidone and vinyl acetate in a ratio of 6:4 by mass (PVPVA64).

Names and abbreviations for polyvinylpyrrolidone-vinyl acetate copolymer include, but are not limited to, PVPVA, PVP-Vac-copolymer, and poly(1-vinylpyrrolidone-co-vinylacetate).

Names and abbreviations for a copolymer of 1-vinyl-2-pyrrolidone and vinyl acetate in a ratio of 6:4 by mass (PVPVA64) include, but are not limited to, copolyvidone, copovidum, and copovidone. Examples of commercially available PVPVA64 are Kollidon^{®} VA64, Kollidon^{®} VA64 Fine, Luviskol VA64^{®}, and Plasdone S-630^{®}.

Names and abbreviations for polyvinylpyrrolidone include, but are not limited to, PVP, povidone and crospovidone. Crospovidone is a crosslinked homopolymer of vinyl pyrrolidone. An example of commercially available PVP is Plasdone^{®} K-12.

Hydroxypropyl methylcellulose, also known as Hypromellose (HPMC) is an anhydroglucose in which some of the hydroxyl groups are substituted with methyl groups to form methyl ether moieties, and others are substituted with hydroxypropyl groups or with methoxypropyl groups to form hydroxypropyl ether or methoxypropyl ether moieties.

Hydroxypropyl methylcellulose polymers (HPMCs) are available in different viscosity grades from several sources such as Dow Chemical Co. under the brand name Methocel^{®} and from Shin Etsu under Metolose^{®}. Examples of low viscosity polymers are Methocel E5^{®}, Methocel E6^{®}, Methocel E-15LV^{®}, Methocel E50LV^{®}, Methocel K100LV^{®} and Methocel F50LV^{®}, whose 2wt% aqueous solutions at 25°C have viscosities of about 5 mPas, 6 mPas, 15 mPas, 50 mPas, 100 mPas and 50 mPas, respectively. Examples of medium viscosity HPMCs are Methocel E4M^{®} and Methocel K4M, whose 2 wt% aqueous solutions at 25°C have viscosities of 4,000 mPas. Examples of high viscosity HPMCs are Methocel K15M^{®} and Methocel K100M^{®} whose 2wt% aqueous solutions at 25°C have viscosities of 15,000 mPas and 100,000 mPas.

In some embodiments, the hydroxypropyl methylcellulose has a viscosity ranging between 3 and 5000 mPa.s in 2 wt% solution in H2O at 25°C such as HPMC E5, HPMC E6, HPMC E15, HPMC E50, HPMC K4M, HPMC K15M, HPMC-AS and mixtures thereof. In some embodiments, the hydroxypropyl methylcellulose has a viscosity ranging between 3 and 500 mPa.s in 2 wt% solution in H2O at 25°C such as HPMC E5, HPMC E6, HPMC E15, HPMC E50, and mixtures thereof. In some embodiments, the hydroxypropyl methylcellulose has a viscosity ranging between 3 and 50 mPa.s in 2 wt% solution in H2O at 25°C such as HPMC E5, HPMC E6, HPMC E15, HPMC E50, and mixtures thereof.

In an embodiment, the invention provides a pharmaceutical formulation, comprising
a) an active pharmaceutical ingredient (API) which is a dengue viral replication inhibitor which is compound of formula (I) as defined herein above and preferably 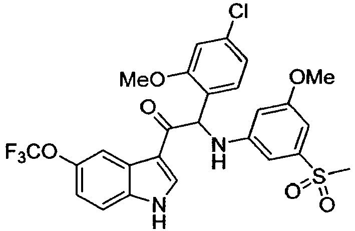 or a stereo-isomeric form, a pharmaceutically acceptable salt, solvate or polymorph thereof; and
b1) methacrylic acid copolymer, or
b2) a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC), or a combination thereof. Preferably, the cellulose derivative is HPMC.

The pharmaceutical formulation of the invention may comprise at most 50 wt%, at most 40 wt%, at most 35 wt%, at most 30 wt%, or at most 25 wt% of the API relative to the total weight of the formulation. The pharmaceutical formulation may comprise at least 0.1 wt%, at least 0.5 wt%, at least 1 wt%, at least 5 wt%, at least 10 wt%, at least 15 wt%, at least 17 wt%, or at least 20 wt% of the API relative to the total weight of the formulation. The pharmaceutical formulation may comprise from 0.1 wt% to 45 wt%, from 0.5 wt% to 40 wt%, from 1 wt% to 40 wt%, from 5 wt% to 35 wt%, or from 10 wt% to 35 wt% of the API relative to the total weight of the formulation.

The pharmaceutical formulation of the invention may contain from 0.1 mg to 3000 mg of the API, from 1 mg to 2000 mg of the API, from 5 mg to 1500 mg of the API, from 5 mg to 1000 mg of the API, from 10 mg to 500 mg of the API, from 15 mg to 400 mg of the API, from 20 mg to 350 mg of the API of the API or any particular amount or range comprised therein. The therapeutically effective amount for said API will vary as will the diseases, syndromes, conditions, and disorders being prevented or treated.

The pharmaceutical formulation of the invention may comprise:
from 20 mg to 6000 mg of the of the methacrylic acid copolymer, from 30 mg to 4000 mg of the methacrylic acid copolymer, from 40 mg to 2000 mg of the methacrylic acid copolymer, from 50 mg to 1500 mg of the methacrylic acid copolymer, from 60 mg to 1000 mg of the methacrylic acid copolymer, from 70 mg to 1000 mg of the methacrylic acid copolymer, from 80 mg to 600 mg of the methacrylic acid copolymer, or any particular amount or range comprised therein; or
from 20 mg to 6000 mg of the of the hydroxypropyl methylcellulose, from 30 mg to 4000 mg of the hydroxypropyl methylcellulose, from 40 mg to 2000 mg of the hydroxypropyl methylcellulose, from 50 mg to 1500 mg of the hydroxypropyl methylcellulose, from 60 mg to 1000 mg of the hydroxypropyl methylcellulose, from 70 mg to 1000 mg of the hydroxypropyl methylcellulose, from 80 mg to 600 mg of the hydroxypropyl methylcellulose or, any particular amount or range comprised therein.

The pharmaceutical formulation of the invention may further comprise one or more diluents; wherein the formulation comprises from 20 mg to 7500 mg of the diluent, preferably from 30 mg to 6500 mg, preferably from 40 mg to 4500 mg, preferably from 50 mg to 2500 mg, preferably from 60 mg to 2000 mg, preferably from 80 mg to 1000 mg, preferably from 90 mg to 550 mg of the diluent, or any particular amount or range comprised therein.

The pharmaceutical formulation of the invention may further comprise one or more surfactants; wherein the formulation comprises from 0.5 mg to 300 mg of the surfactant, preferably from 0.6 mg to 250 mg, preferably from 0.8 mg to 200 mg, preferably from 1 mg to 150 mg, preferably from 1.2 mg to 100 mg, preferably from 1.5 mg to 80 mg, preferably from 2 mg to 30 mg of the surfactant, or any particular amount or range comprised therein.

The pharmaceutical formulation of the invention may further comprise one or more disintegrant; wherein the formulation comprises from 3 mg to 900 mg of the disintegrant, preferably from 4 mg to 850 mg, preferably from 5 mg to 600 mg, preferably from 6 mg to 500 mg, preferably from 7 mg to 400 mg, preferably from 7.5 mg to 200 mg, preferably from 8 mg to 100 mg of the disintegrant, or any particular amount or range comprised therein.

The pharmaceutical formulation of the invention may further comprise one or more glidant; wherein the formulation comprises from 1 mg to 400 mg of the glidant, preferably from 2 mg to 350 mg, preferably from 3 mg to 300 mg, preferably from 4 mg to 200 mg, preferably from 5 mg to 100 mg, preferably from 6 mg to 50 mg, preferably from 8.5 mg to 35 mg of the glidant, or any particular amount or range comprised therein.

The pharmaceutical formulation of the invention may further comprise one or more lubricant; wherein the formulation comprises from 0.5 mg to 200 mg of the lubricant, preferably from 1 mg to 150 mg, preferably from 3 mg to 100 mg, preferably from 4 mg to 90 mg, preferably from 7 mg to 90 mg, preferably from 8 mg to 80 mg, preferably from 10 mg to 50 mg of the lubricant, or any particular amount or range comprised therein.

The pharmaceutical formulation of the invention may comprise:
at most 60 wt%, most 50 wt%, at most 45 wt%, at most 40 wt%, or at most 35 wt% of the methacrylic acid copolymer relative to the total weight of the formulation; or
at most 60 wt%, most 50 wt%, at most 45 wt%, at most 40 wt%, or at most 35 wt% of the hydroxypropyl methylcellulose relative to the total weight of the formulation.

The pharmaceutical formulation of the invention may comprise:
at least 0.2 wt%, at least 1 wt%, at least 5 wt%, at least 10 wt%, at least 20 wt%, of the methacrylic acid copolymer relative to the total weight of the formulation; or
at least 0.2 wt%, at least 1 wt%, at least 5 wt%, at least 10 wt%, at least 20 wt%, of the hydroxypropyl methylcellulose relative to the total weight of the formulation.

The pharmaceutical formulation may comprise:
from 0.2 wt% to 60 wt%, from 1 wt% to 50 wt%, or from 5 wt% to 40 wt% of the methacrylic acid copolymer relative to the total weight of the formulation; or
from 0.2 wt% to 60 wt%, from 1 wt% to 50 wt%, or from 5 wt% to 40 wt% of the hydroxypropyl methylcellulose relative to the total weight of the formulation.

In some embodiments, the API and the methacrylic acid copolymer are present in the pharmaceutical formulation of the invention in a ratio of 4:1 w/w; a ratio of 3.8:1 w/w; a ratio of 3.5:1 w/w; a ratio of 3.3:1 w/w; a ratio of 3:1 w/w; a ratio of 2.8:1 w/w; a ratio of 2.5:1 w/w; a ratio of 2.3:1 w/w; a ratio of 2:1 w/w; a ratio of 1.8:1 w/w; a ratio of 1.5:1 w/w; a ratio of 1:5 w/w; a ratio of 1:4.8 w/w; a ratio of 1:4.5 w/w; a ratio of 1:4.3 w/w; a ratio of 1:4 w/w; a ratio of 1:3.8 w/w; a ratio of 1:3.5 w/w; a ratio of 1:3.3 w/w; a ratio of 1:3 w/w; a ratio of 1:2.8 w/w; a ratio of 1:2.5 w/w; a ratio of 1:2.3 w/w; a ratio of 1:2 w/w; a ratio of 1:1.5 w/w or a ratio of 1:1.2 w/w or a ratio comprised within any two ratios mentioned herein, or a ratio range or sub-range within any two ratios mentioned herein.

In some embodiments, the API and the hydroxypropyl methylcellulose are present in the pharmaceutical formulation of the invention in a ratio of 4:1 w/w; a ratio of 3.8:1 w/w; a ratio of 3.5:1 w/w; a ratio of 3.3:1 w/w; a ratio of 3:1 w/w; a ratio of 2.8:1 w/w; a ratio of 2.5:1 w/w; a ratio of 2.3:1 w/w; a ratio of 2:1 w/w; a ratio of 1.8:1 w/w; a ratio of 1.5:1 w/w; a ratio of 1:5 w/w; a ratio of 1:4.8 w/w; a ratio of 1:4.5 w/w; a ratio of 1:4.3 w/w; a ratio of 1:4 w/w; a ratio of 1:3.8 w/w; a ratio of 1:3.5 w/w; a ratio of 1:3.3 w/w; a ratio of 1:3 w/w; a ratio of 1:2.8 w/w; a ratio of 1:2.5 w/w; a ratio of 1:2.3 w/w; a ratio of 1:2 w/w; a ratio of 1:1.5 w/w or a ratio of 1:1.2 w/w or a ratio comprised within any two ratios mentioned herein, or a ratio range or sub-range within any two ratios mentioned herein.

The pharmaceutical formulation of the invention may further comprise one or more pharmaceutically acceptable excipients, as described in more detail herein. Pharmaceutically acceptable excipients include, but are not limited to, disintegrants, binders, diluents, lubricants, stabilizers, osmotic agents, colorants, plasticizers, coatings and the like. Additional suitable pharmaceutical excipients and their properties may be found in texts such as Handbook of Pharmaceutical Excipients, Edited by R.C. Rowe, P.J. Sheskey & P.J. Weller, Sixth Edition (Published by Pharmaceutical Press, a Division of Royal Pharmaceutical Society of Great Britain).

### Diluents/Fillers.

Diluents (or fillers) useful in the present invention include microcrystalline celluloses (e.g., Avicel^{®} PH 102, Avicel^{®} PH 101, Ceolus UF, Ceolus KG, or Ceolus PH), silicified microcrystalline celluloses, lactoses, sorbitols, celluloses, calcium phosphates, starches (e.g., partially or fully pregelatinized maize starch), sugars or lactoses (e.g., mannitol, sucrose, or the like), or any combination thereof. Examples of microcrystalline celluloses include commercially available Avicel^{®} series, such as microcrystalline celluloses having a particle size of 100 µm (e.g., Avicel^{®} PH 102). Microcrystalline celluloses also include commercially available Ceolus in UF, KG, or PH grade. Other examples of diluents include silicified microcrystalline celluloses, such as commercially available Prosolv^{®} series (e.g., Prosolv^{®} SMCC 50 and SMCC HD90). Lactoses suitable for the invention includes lactose monohydrate. Amounts of the diluents relative to the total weight of the pharmaceutical formulation may be 5 wt% to 95 wt%, 20 wt% to 80 wt%, 25 wt% to 50 wt%, 30 wt% to 48 wt%, 30 wt % to 52 wt%, 35 wt% to 52 wt% or 40 wt% to 50 wt%. For example, the diluent in the pharmaceutical formulation may comprise microcrystalline cellulose, silicified microcrystalline cellulose, and partially or fully pregelatinized maize starch having a combined (or total) concentration of 5 wt% to 95 wt%, 20 wt% to 80 wt%, 25 wt% to 50 wt%, 30 wt% to 48 wt%, 30 wt % to 52 wt%, 35 wt% to 52 wt%, 30 wt% to 45 wt% or 32.5 wt% to 45 wt% by weight of the pharmaceutical formulation.

### Disintegrants.

Disintegrants enhance the dispersal of pharmaceutical formulations. Non-limiting examples of disintegrants that are useful in the present invention include croscarmelloses (e.g., croscarmellose sodium), crospovidone, metal starch glycolates (e.g., sodium starch glycolate), and any combination thereof. Other examples of disintegrants include croscarmellose sodium (e.g., Ac-Di-Sol^{®}) and sodium starch glycolate. Pharmaceutical formulations of the present invention may comprise one or more disintegrants giving a combined (or total) concentration of 1 wt% to 10 wt%, 5 wt% to 9 wt%, 6 wt% to 8 wt%, 6.5 wt% to 7.5 wt%, 6.75 wt% to 7.25 wt%, 3 wt% to 7 wt%, 1 wt% to 7 wt%, or 1.2 wt% to 8.2 wt% of the pharmaceutical formulation. In some embodiments, the pharmaceutical formulation comprises 2 wt% to 8 wt% (e.g., 2.5 wt% to 7.5 wt%), preferably from 3 wt% to 6 wt%; more preferably from 4 wt% to 5 wt% of disintegrant (e.g., crospovidone) by weight of the pharmaceutical formulation.

### Binders.

Binders may include agents used while making granules of the active pharmaceutical ingredient by mixing the binder(s) with diluent and the active pharmaceutical ingredient. Non-limiting examples of binders useful in the present invention include polyvinyl pyrrolidones, sugar, modified celluloses (e.g., hydroxypropyl methylcelluloses (HPMC), hydroxy propyl celluloses (HPC), and hydroxy ethyl celluloses (HEC)), and any combination thereof. Other examples of the binders include polyvinyl pyrrolidones (PVP). An example of HPC includes a low viscosity polymer, HPC-SL. PVP may be characterized by its "K-value", which is a useful measure of the polymeric composition's viscosity. PVP can be commercially purchased (e.g., Tokyo Chemical Industry Co., Ltd.) under the trade name of Povidone^{®} K12, Povidone^{®} K17, Povidone^{®} K25, Povidone^{®} K30, Povidone^{®} K60, and Povidone^{®} K90. Specific examples of PVP include soluble spray dried PVP. Another example includes PVP having an average molecular weight of 3,000 to 4,000, such as Povidone^{®} K12 having an average molecular weight of 4,000. PVP can be used in either wet or dry state. Pharmaceutical formulations of the present invention may comprise one or more binders giving a combined (or total) concentration of 0.1 wt% to 50 wt%; 0.5 wt% to 43 wt%; 2 wt% to 45 wt%; 5 wt% to 40 wt%, 10 wt% to 35 wt%, 15 wt% to 30 wt%; 20 wt% to 25 wt% of the pharmaceutical formulation. In some embodiments, the pharmaceutical formulation comprises 0.5 wt% to 2 wt% (e.g., 1.5 wt% to 2.0 wt% or 1.75 wt% to 2.25 wt%) of binder (e.g., hydroxypropyl methylcellulose) by weight of the pharmaceutical formulation.

### Lubricants.

Lubricants function to improve the compression and ejection of pharmaceutical formulations from, e.g., a die press. Non-limiting examples of lubricants useful in the present invention include magnesium stearate, stearic acid (stearin), hydrogenated oil, sodium stearyl fumarate, compritol (glyceryl behenate) and any combination thereof. In one example, the lubricant includes sodium stearyl fumarate. In another example, the lubricant includes magnesium stearate. Pharmaceutical formulations of the present invention may comprise one or more lubricants giving a combined (or total) concentration of 0.1 wt% to 10 wt%, 0.5 wt% to 6 wt%, 0.8 wt% to 3.5 wt%, 1 wt% to 3 wt%, 1.5 wt% to 5.5 wt%, 2 wt% to 4 wt% or 0.25 wt% to 5.25 wt% by weight of the pharmaceutical formulation. In some embodiments, the pharmaceutical formulation comprises 0.5 wt% to 5.5 wt%, preferably 1 wt% to 2.5 wt% of lubricant (e.g., magnesium stearate).

### Wetting Agents/Surfactants.

One or more wetting agents can be employed in the pharmaceutical formulations of the invention. Wetting agents suitable for the present invention generally enhance the solubility of pharmaceutical formulations. Wetting agents include surfactants, such as non-ionic surfactants and anionic surfactants. Non-limiting examples of surfactants useful in the invention include sodium lauryl sulfate (SLS), polyoxyethylene sorbitan fatty acids (e.g., polysorbate 20 (e.g., TWEEN 20^{™})), sorbitan fatty acid esters (e.g., Spans^{®}), sodium dodecylbenzene sulfonate (SDBS), dioctyl sodium sulfosuccinate (Docusate), dioxycholic acid sodium salt (DOSS), sorbitan monostearate, sorbitan tristearate, sodium N-lauroylsarcosine, sodium oleate, sodium myristate, sodium stearate, sodium palmitate, gelucire 44/14, ethylenediamine tetraacetic acid (EDTA), vitamin E d-alpha tocopheryl polyethylene glycol 1000 succinate (TPGS), lecithin, MW 677-692, glutanic acid monosodium monohydrate, labrasol, PEG 8 caprylic/capric glycerides, transcutol, diethylene glycol monoethyl ether, solutol HS-15, Soluplus^{®}, polyethylene glycol/hydroxystearate, taurocholic acid, copolymers of polyoxypropylene and polyoxyethylene (e.g., poloxamers also known and commercially available under pluronics^{®}, such as, pluronic^{®} L61, pluronic^{®} F68, pluronic^{®} F108, and pluronic^{®} F127), saturated polyglycolized glycerides (gelucirs^{®}), and any combination thereof. Other examples include sodium lauryl sulfate, which is an anionic surfactant; and copolymers of polyoxypropylene and polyoxyethylene which are non-ionic surfactants. Examples of the copolymers of polyoxypropylene and polyoxyethylene include poloxamers, such as poloxamer with a polyoxypropylene molecular mass of 1,800 g/mol and an 80% polyoxyethylene content (e.g., poloxamer 188). Pharmaceutical formulations of the present invention may comprise one or more wetting agents giving a combined (or total) concentration of 0.25 wt% to 10 wt%, 0.25 wt% to 5.75 wt %, 0.5 wt% to 5 wt%, 1 wt% to 3 wt% or 1.5 wt% to 2 wt% by weight of the pharmaceutical formulation. In some embodiments, the pharmaceutical formulation comprises 0.25 wt% to 5 wt% (e.g., 0.35 wt% to 4.5 wt%) of a wetting agent (e.g., sodium lauryl sulfate).

### Glidants.

Glidants enhance the flow properties of formulations during processing into final drug product form. Non-limiting examples of glidants useful in the present invention include silicon dioxide (e.g., colloidal fumed silica, colloidal anhydrous silica) and/or talc. Specific examples of glidants include colloidal fumed silica (e.g., Aerosil^{®} 200) Pharmaceutical formulations of the present invention may comprise one or more glidants giving a combined (or total) concentration of 0.1 wt% to 10 wt%, preferably 1 wt% to 8 wt%; preferably 2 wt% to 7.5 wt%; preferably 3 wt% to 5 wt% by the weight of the pharmaceutical formulation. In some embodiments, the pharmaceutical formulation comprises glidant in an amount of 0.10 wt% to 5 wt% (e.g., 0.75 wt% to 3.25 wt%) by weight of the pharmaceutical formulation. In other embodiments, the pharmaceutical formulation comprises fumed silica in an amount of 0.10 wt% to 2 wt% (e.g., 0.75 wt% to 1.5 wt%) by weight of the pharmaceutical formulation.

Tablet dosage forms may further comprise a coating. Suitable coatings are film-forming polymers, such as, for example, those from the group of the cellulose derivatives (such as HPC (hydroxypropylcellulose), HPMC (hydroxypropoxymethylcellulose), MC (methylcellulose), HPMCAS (hydroxypropoxymethylcelluclose acetate succinate), dextrins, starches, opdary^{®}, natural gums, such as, for example, gum arabic, xanthans, alginates, polyvinyl alcohol, polymethacrylates and derivatives thereof, such as, for example, Eudragit^{®}, which may be applied to the tablet as solutions or suspensions by means of the various pharmaceutical conventional methods, such as, for example, film coating. The coating is typically applied as a solution/suspension which, in addition to any film-forming polymer present, may further comprise one or more adjuvants, such as hydrophilisers, plasticisers, surfactants, dyes and white pigments, such as, for example, titanium dioxide.

In some embodiments the pharmaceutical formulation according to the invention comprises a plurality of granules forming an intragranular phase of the formulation and one or more excipients forming an extragranular phase of the formulation. Preferably the pharmaceutical formulation comprises a tablet, said tablet comprising an intragranular phase and an extragranular phase.

As used herein, the term "intragranular phase" refers to those components of a formulation that are with granules and/or within granules. As used herein, the term "extragranular phase" refers to those components of a formulation that are outside of the granules.

In some embodiments the intragranular phase of the pharmaceutical formulation according to the invention comprises the active pharmaceutical ingredient and one of or a combination of methacrylic acid copolymer, or a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC). In some embodiments the intragranular phase of the pharmaceutical formulation according to the invention comprises the active pharmaceutical ingredient and one of or a combination of methacrylic acid copolymer, or hydroxypropyl methylcellulose (HPMC) and one or more excipients.

In some embodiments the pharmaceutical formulation comprises at least 15 wt%; at least 20 wt%; at least 25 wt%; at least 28 wt%; at least 30 wt%; at least 34 wt%; at least 40 wt%; at least 45 wt%, at least 50 wt%; at least 55 wt%; at least 60 wt%; at least 65 wt% of intragranular phase by the total weight of the pharmaceutical formulation. In some embodiments the pharmaceutical formulation comprises at most 99 wt%; at most 95 wt%; at most 93 wt%; at most 90 wt%; at most 85 wt%; at most 80 wt%; at most 75 wt%; at most 74 wt%; at most 73 wt%; at most 70 wt%; at most 67 wt%; at most 63 wt%; at most 60% wt%; at most 53 wt% of intragranular phase by the total weight of the pharmaceutical formulation.

In some embodiments the pharmaceutical formulation comprises an intragranular phase comprising from preferably from 1 wt% to 70 wt%; preferably from 2 wt% to 69 wt%; preferably from 3 wt% to 68 wt%; preferably from 4 wt% to 67 wt%; preferably from 5 wt% to 66 wt%; preferably from 6 wt% to 65 wt%; preferably from 10 wt% to 64 wt%; preferably from 15 wt% to 60 wt%,; preferably from 20 wt% to 55 wt%; preferably from 25 wt% to 50 wt%; preferably from 30 wt% to 45 wt%; preferably from 35 to 40 wt% of API by the total weight of the pharmaceutical formulation.

In some embodiments the pharmaceutical formulation comprises an intragranular phase comprising from 5 wt% to 60 wt%; preferably from 7 wt% to 55 wt%; preferably from 8 wt% to 50 wt%; preferably from 17 wt% to 45 wt%; preferably from 15 to 45 wt% of methacrylic acid copolymer or hydroxypropyl methylcellulose or a combination thereof by the total weight of the pharmaceutical formulation.

In some embodiments the pharmaceutical formulation comprises an intragranular phase comprising from 5 wt% to 60 wt%; preferably from 10 wt% to 60 wt%; preferably from 12 wt% to 60 wt%; preferably from 15 wt% to 50 wt%; preferably from 18 wt% to 45 wt% of filler by the total weight of the pharmaceutical formulation.

In some embodiments the pharmaceutical formulation comprises an intragranular phase comprising from 1 wt% to 10 wt%; preferably from 1 wt% to 9 wt%, preferably from 1.7 wt% to 8 wt%; preferably from 1.6 wt% to 7.5 wt%; preferably from 2 wt% to 5 wt% of disintegrant by the total weight of the pharmaceutical formulation.

In some embodiments the pharmaceutical formulation comprises an intragranular phase comprising from 0.1 wt% to 5 wt%; preferably from 0.2 wt% to 4.7 wt%; preferably from 0.5 wt% to 4.5 wt%; preferably from 0.8 wt% to 4 wt%; preferably from 1 wt% to 3.5 wt% of glidant by the total weight of the pharmaceutical formulation.

In some embodiments the pharmaceutical formulation comprises an intragranular phase comprising from 0.1 wt% to 5 wt%; preferably from 0.2 wt% to 4.7 wt%; preferably from 0.5 wt% to 4.5 wt%; preferably from 0.6 wt% to 4.5 wt%; preferably from 0.8 wt% to 4.0 wt%; preferably from 1 wt% to 3.5 wt% of surfactant by the total weight of the pharmaceutical formulation
In some embodiments the pharmaceutical formulation comprises an intragranular phase comprising from 0.1 wt% to 3 wt%; preferably from 0.2 wt% to 2.7 wt%; preferably from 0.5 wt% to 2.5 wt%; preferably from 0.7 wt% to 2 wt% of lubricant by the total weight of the pharmaceutical formulation.

In some embodiments the pharmaceutical formulation comprises at least 5wt%; at least 7 wt%; at least 10 wt%; at least 15 wt%; at least 20 wt%; at least 25 wt%; at least 28 wt%; at least 30 wt%; at least 34 wt%; at least 35 wt% of extragranular phase by the total weight of the pharmaceutical formulation. In some embodiments the pharmaceutical formulation comprises at most 75 wt%; at most 74 wt%; at most 73 wt%; at most 70 wt%; at most 67 wt%; at most 63 wt%; at most 65 wt%; at most 60 wt%; at most 55 wt% at most 53 wt%, at most 40 wt% of extragranular phase by the total weight of the pharmaceutical formulation. In some embodiments the pharmaceutical formulation comprises at least 15 wt% to at most 75 wt%; at least 25 wt% to at most 70 wt%; at least 30 wt% to at most 65 wt%; at least 35 wt% to at most 60 wt%; at least 35 wt% to at most 70 wt% of extragranular phase by the total weight of the pharmaceutical formulation.

In some embodiments the pharmaceutical formulation comprises at least 15 wt%; at least 20 wt%; at least 25 wt%; at least 28 wt%; at least 30 wt%; at least 34 wt%; at least 35 wt% of intragranular phase by the total weight of the pharmaceutical formulation. In some embodiments the pharmaceutical formulation comprises at most at most 99 wt%; at most 93 wt%; at most 85 wt%; at most 80 wt%; at most 75 wt%; at most 74 wt%; at most 73 wt%; at most 70 wt%; at most 67 wt%; at most 65 wt%; at most 63 wt%; at most 60 wt%; at most 55 wt%; at most 53 wt% of intragranular phase by the total weight of the pharmaceutical formulation. In some embodiments the pharmaceutical formulation comprises at least 15 wt% to at most 75 wt%; at least 25 wt% to at most 70 wt%; at least 30 wt% to at most 65 wt%; at least 35 wt% to at most 60 wt%; at least 35 wt% to at most 70 wt% of intragranular phase by the total weight of the pharmaceutical formulation.

In some embodiments the pharmaceutical formulation comprises an intragranular phase comprising:
(i) from 5 wt% to 45 wt%, from 10 wt% to 40 wt%, from 15 wt% to 35 wt% of API by the total weight of the pharmaceutical formulation;
(ii) from 5 wt% to 60 wt%, from 10 wt% to 50 wt%, from 15 wt% to 55 wt% of methacrylic acid copolymer or hydroxypropyl methylcellulose or a combination thereof by the total weight of the pharmaceutical formulation;
(iii) from 5 wt% to 60 wt%, from 10 wt% to 50 wt%, from 15 wt% to 40 wt% of
   filler (e.g., mannitol, microcrystalline cellulose) by the total weight of the pharmaceutical formulation;
(iv) from 1 wt% to 10 wt%, from 1.5 wt% to 9 wt%, from 1.5 wt% to 8 wt% of disintegrant (e.g., croscarmellose sodium) by the total weight of the pharmaceutical formulation;
(v) from 0.1 wt% to 5 wt%, from 0.2 wt% to 4.5 wt%, from 0.5 wt% to 4 wt% of glidant (e.g., colloidal fumed silica) by the total weight of the pharmaceutical formulation;
(vi) from 0.1 wt% to 5 wt%, from 0.2 wt% to 4.5 wt%, from 0.5 wt% to 4 wt% of surfactant (e.g., sodium lauryl sulfate) by the total weight of the pharmaceutical formulation; and
(vii) from 0.1 wt% to 3 wt%, from 0.2 wt% to 2.5 wt%, from 0.5 wt% to 2 wt% of lubricant (e.g., magnesium stearate) by the total weight of the pharmaceutical formulation.

In some embodiments the pharmaceutical formulation comprises an extragranular phase comprising from 0.1 wt% to 5.0 wt%; preferably from 0.2 wt% to 4.7 wt%; preferably from 0.5 wt% to 4.5 wt%; preferably from 1 wt% to 3.5 wt%; preferably from 1.5 wt% to 3 wt% of disintegrant by the total weight of the pharmaceutical formulation.

In some embodiments the pharmaceutical formulation comprises an extragranular phase comprising from 0.1 wt% to 3 wt%; preferably from 0.2 wt% to 2.7 wt%; preferably from 0.5 wt% to 2.5 wt% ; preferably from 0.8 wt% to 2 wt% of lubricant by the total weight of the pharmaceutical formulation.

In some embodiments the pharmaceutical formulation comprises an extragranular phase comprising from 0.1 wt% to 55 wt%; from 0.2 wt% to 50 wt%; from 0.3 wt% to 45 wt%; from 0.4 wt% to 40 wt%; from 0.5 wt% to 35 wt%; from 0.6 wt% to 30 wt%; from 0.7 wt% to 25 wt%; from 0.8 wt% to 20 wt%; 1 wt% to 15 wt%; from 1.3 wt% to 12 wt%; from 2 wt% to 10 wt%; from 2.5 wt% to 9 wt%; from 3 wt% to 8 wt%; from 4 wt% to 7 wt%; from 5 wt% to 6 wt% of filler by the total weight of the pharmaceutical formulation.

In some embodiments the pharmaceutical formulation comprises an extragranular phase comprising:
(a) from 0.1 wt% to 5.0 wt%, from 0.2 wt% to 4.5 wt%, from 0.5 wt% to 3 wt% of disintegrant (e.g., croscarmellose sodium) by the total weight of the pharmaceutical formulation;
(b) from 0.1 wt% to 3 wt%, from 0.2 wt% to 2.5 wt% or from 0.5 wt% to 2 wt% of lubricant (e.g. magnesium stearate) by the total weight of the pharmaceutical formulation; and
(c) from 1 wt% to 15 wt%, from 1.5 wt% to 10 wt% or from 2 wt% to 8 wt% of filler (e.g., hydroxypropyl methylcellulose) by the total weight of the pharmaceutical formulation.

In some embodiments, the pharmaceutical formulation of the invention comprises an intragranular phase and an extragranular phase; wherein the formulation comprises from 50 mg to 17000 mg of the intragranular phase, preferably from 80 mg to 10000 mg, preferably from 100 mg to 5000 mg, preferably from 120 mg to 3000 mg, preferably from 150 mg to 2500 mg, preferably from 200 mg to 2000 mg, preferably from 250 mg to 1200 mg of the intragranular phase, or any particular amount or range comprised therein.

In some embodiments, the pharmaceutical formulation of the invention comprises an intragranular phase and an extragranular phase; wherein the formulation comprises from 4 mg to 1500 mg of the extragranular phase, preferably from 6 mg to 1000 mg, preferably from 8 mg to 500 mg, preferably from 10 mg to 300 mg, preferably from 15 mg to 250 mg, preferably from 20 mg to 200 mg, preferably from 30 mg to 100 mg of the extragranular phase, or any particular amount or range comprised therein.

In some embodiments the pharmaceutical formulation comprises an intragranular phase comprising from 1 mg to 2000 mg; preferably from 5 mg to 1500 mg; preferably; from 5 mg to 1000 mg; preferably from 10 mg to 500 mg; preferably from 15 mg to 400 mg; preferably from 20 mg to 350 mg of API, or any particular amount or range comprised therein.

In some embodiments the pharmaceutical formulation comprises an intragranular phase comprising from 20 mg to 6000; preferably 30 mg to 4000 mg; preferably from 40 mg to 2000 mg; preferably from 70 mg to 1000 mg of one of methacrylic acid copolymer or hydroxypropyl methylcellulose or a combination thereof, or any particular amount or range comprised therein.

In some embodiments the pharmaceutical formulation comprises an intragranular phase comprising from 10 mg to 6500 mg, preferably from 12 mg to 5000 mg, preferably from 15 mg to 4000 mg, preferably from 15 mg to 2000 mg, preferably from 18 mg to 1000 mg, preferably from 18 mg to 500 mg of diluent/filler, or any particular amount or range comprised therein.

In some embodiments the pharmaceutical formulation comprises an intragranular phase comprising from 0.5 mg to 300 mg, preferably from 0.6 mg to 250 mg, preferably from 0.8 mg to 200 mg, preferably from 1 mg to 150 mg, preferably from 1.2 mg to 100 mg, preferably from 1.5 mg to 80 mg, preferably from 2 mg to 30 mg of surfactant, or any particular amount or range comprised therein.

In some embodiments the pharmaceutical formulation comprises an intragranular phase comprising from 2 mg to 720 mg, preferably from 4 mg to 650 mg, preferably from 5 mg to 400 mg, preferably from 6 mg to 300 mg, preferably from 7 mg to 200 mg, preferably from 7.5 mg to 100 mg, preferably from 8 mg to 60 mg of disintegrant, or any particular amount or range comprised therein.

In some embodiments the pharmaceutical formulation comprises an intragranular phase comprising from 1 mg to 400 mg, preferably from 2 mg to 350 mg, preferably from 3 mg to 300 mg, preferably from 4 mg to 200 mg, preferably from 5 mg to 100 mg, preferably from 6 mg to 50 mg, preferably from 8.5 mg to 35 mg of glidant, or any particular amount or range comprised therein.

In some embodiments the pharmaceutical formulation comprises an intragranular phase comprising from 0.3 mg to 90 mg; preferably from 0.5 mg to 70 mg; preferably from 0.6 mg to 50 mg; preferably from 0.7 mg to 25 mg; preferably from 0.8 mg to 10 mg of lubricant, or any particular amount or range comprised therein.

In some embodiments the pharmaceutical formulation comprises an intragranular phase comprising:
(i) from 1 mg to 2000 mg; preferably from 5 mg to 1500 mg; preferably; from 5 mg to 1000 mg; preferably from 10 mg to 500 mg; preferably from 15 mg to 400 mg; preferably from 20 mg to 350 mg of API;
(ii) from 20 mg to 6000; preferably 30 mg to 4000 mg; preferably from 40 mg to 2000 mg; preferably from 70 mg to 1000 mg of one of methacrylic acid copolymer or hydroxypropyl methylcellulose or a combination thereof;
(iii) from 10 mg to 6500 mg, preferably from 12 mg to 5000 mg, preferably from 15 mg to 4000 mg, preferably from 15 mg to 2000 mg, preferably from 18 mg to 1000 mg, preferably from 18 mg to 500 mg of diluent/filler (e.g., mannitol, microcrystalline cellulose);
(iv) from 2 mg to 720 mg, preferably from 4 mg to 650 mg, preferably from 5 mg to 400 mg, preferably from 6 mg to 300 mg, preferably from 7 mg to 200 mg, preferably from 7.5 mg to 100 mg, preferably from 8 mg to 60 mg of disintegrant (e.g., croscarmellose sodium);
(v) from 1 mg to 400 mg, preferably from 2 mg to 350 mg, preferably from 3 mg to 300 mg, preferably from 4 mg to 200 mg, preferably from 5 mg to 100 mg, preferably from 6 mg to 50 mg, preferably from 8.5 mg to 35 mg of glidant (e.g., colloidal fumed silica);
(vi) from 0.5 mg to 300 mg, preferably from 0.6 mg to 250 mg, preferably from 0.8 mg to 200 mg, preferably from 1 mg to 150 mg, preferably from 1.2 mg to 100 mg, preferably from 1.5 mg to 80 mg, preferably from 2 mg to 30 mg of surfactant (e.g., sodium lauryl sulfate); and
(vii) from 0.3 mg to 90 mg; preferably from 0.5 mg to 70 mg; preferably from 0.6 mg to 50 mg; preferably from 0.7 mg to 25 mg; preferably from 0.8 mg to 10 mg of lubricant (e.g., magnesium stearate).

In some embodiments the pharmaceutical formulation comprises an extragranular phase comprising from 0.6 mg to 180 mg; preferably from 1 mg to 100 mg; preferably from 2 mg to 80 mg; preferably from 3 mg to 50 mg; preferably from 5 mg to 20 mg of disintegrant.

In some embodiments the pharmaceutical formulation comprises an extragranular phase comprising from 3.5 mg to 1100 mg; preferably from 5 mg to 500 mg; preferably from 10 mg to 250 mg; preferably from 15 mg to 100 mg; preferably from 25 mg to 90 mg of diluent/filler.

In some embodiments the pharmaceutical formulation comprises an extragranular phase comprising from 0.3 mg to 90 mg; preferably from 0.5 mg to 70 mg; preferably from 1 mg to 50 mg; preferably from 1.5 mg to 20 mg; preferably from 2 mg to 10 mg of lubricant.

In some embodiments the pharmaceutical formulation comprises an extragranular phase comprising:
(a) from 0.6 mg to 180 mg; preferably from 1 mg to 100 mg; preferably from 2 mg to 80 mg; preferably from 3 mg to 50 mg; preferably from 5 mg to 20 mg of disintegrant (e.g., croscarmellose sodium);
(b) from 0.3 mg to 90 mg; preferably from 0.5 mg to 70 mg; preferably from 1 mg to 50 mg; preferably from 1.5 mg to 20 mg; preferably from 2 mg to 10 mg of lubricant (e.g. magnesium stearate); and
(c) from 3.5 mg to 1100 mg; preferably from 5 mg to 500 mg; preferably from 10 mg to 250 mg; preferably from 15 mg to 100 mg; preferably from 25 mg to 90 mg of diluent/filler (e.g., hydroxypropyl methylcellulose).

One skilled in the art will readily recognize that the appropriate pharmaceutically acceptable excipients are selected such that they are compatible with other excipients and do not bind or interact with the active pharmaceutical ingredient or cause degradation of the active ingredient or of the pharmaceutical formulation.

It will be appreciated that any of the above description relating to components of the pharmaceutical formulation may apply to any of the other aspects and embodiments of the invention.

### Active Pharmaceutical Ingredient

Suitable active pharmaceutical ingredients (API) are those which exert a pharmacological, immunological or metabolic action with a view to restoring, correcting or modifying physiological functions or to make a medical diagnosis. Non-limiting examples thereof include analgesic and anti-inflammatory drugs; anti-arrhythmic drugs; antibacterial and antiprotozoal agents; anti-coagulants; antidepressants; anti-diabetic drugs; anti-epileptic drugs; antifungal agents; antihistamines; anti-hypertensive drugs; anti-muscarinic agents; antineoplastic agents and antimetabolites; anti-migraine drugs; anti-Parkinsonian drugs; antipsychotic, hypnotic and sedating agents; anti-stroke agents; antitussive; antivirals; beta-adrenoceptor blocking; cardiac inotropic agents; corticosteroids; disinfectants; diuretics; enzymes; essential oils; gastro-intestinal agents; lipid regulating agents; local anaesthetics; opioid analgesics; parasympathomimetics and anti-dementia drugs; sex hormones; stimulating agents and vasodilators.

The invention provides a pharmaceutical formulation, comprising
a) an API; and
b1) methacrylic acid copolymer, or
b2) a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC), or a combination thereof;
wherein the API is a dengue viral replication inhibitor.

In particular, the API is in amorphous form or dissolved state (i.e. molecular dispersion) in the pharmaceutical formulation.

In an embodiment, the API is a dengue viral replication inhibitor. Embodiments of the invention include a pharmaceutical formulation as described herein, wherein the API is a compound of Formula (I) 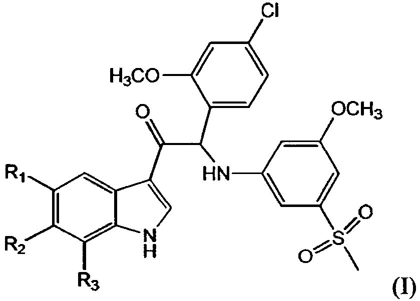 a stereo-isomeric form, a pharmaceutically acceptable salt, solvate or polymorph thereof; said compound is selected from the group wherein:
R₁ is H, R₂ is F and R₃ is H or CH₃,
R₁ is H, CH₃ or F, R₂ is OCH₃ and R₃ is H and
R₁ is H, R₂ is OCH₃ and R₃ is CH₃,
R₁ is CH₃, R₂ is F and R₃ is H,
R₁ is CF₃ or OCF₃, R₂ is H and R₃ is H,
R₁ is OCF₃, R₂ is OCH₃ and R₃ is H and
R₁ is OCF₃, R₂ is H and R₃ is CH₃.

Additional embodiments of the invention include pharmaceutical formulations as described herein, wherein the API is a compound of Formula (I) selected from the group consisting of: 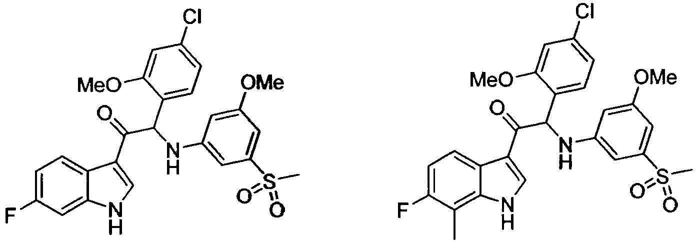 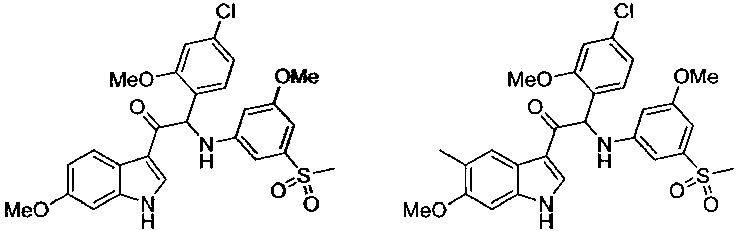 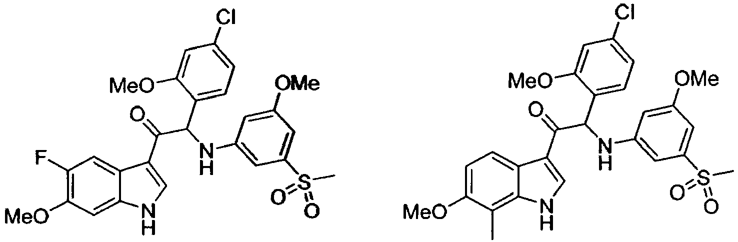 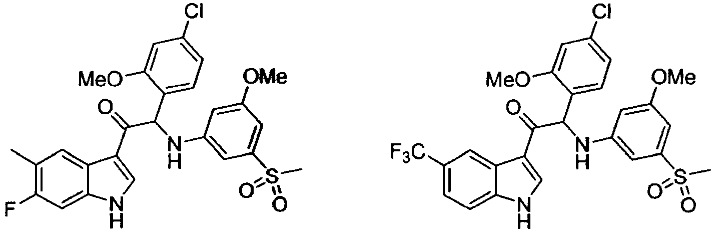 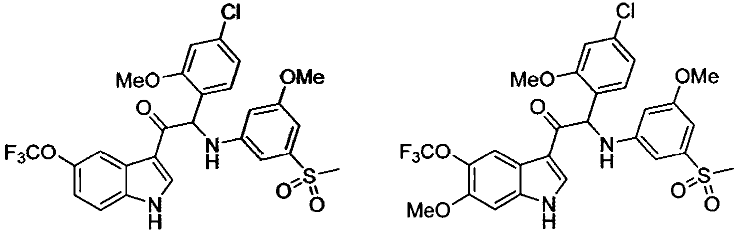 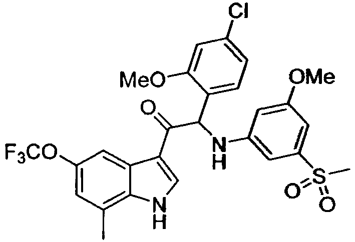 or its stereo-isomeric form, a pharmaceutically acceptable salt, solvate or polymorph thereof.

In particular, the API is a compound of Formula (I), or an enantiomer, diastereomer or pharmaceutically acceptable salt form thereof.

In particular, the API is a compound of Formula (I), or an enantiomer, diastereomer or pharmaceutically acceptable salt form thereof, in amorphous state or dissolved state (i.e. molecular dispersion).

In particular, the API used as starting material in the process to prepare a pharmaceutical formulation as described herein, is a compound of Formula (I), or an enantiomer, diastereomer, solvate, or a pharmaceutically acceptable salt form thereof; while the API in the final pharmaceutical formulation or solid dosage form as defined herein is a compound of Formula (I), or an enantiomer, diastereomer, or pharmaceutically acceptable salt form thereof, in amorphous form or dissolved state.

In a preferred embodiment, the compound of Formula (I) is 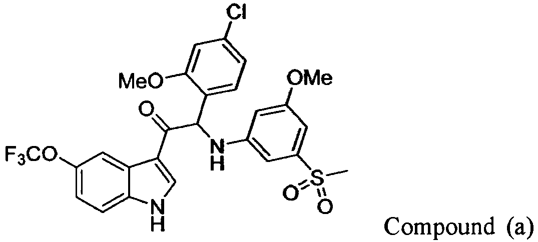 or a stereo-isomeric form, a pharmaceutically acceptable salt, solvate or polymorph thereof.

The API may be Compound (a) or a solvate or pharmaceutically acceptable salt form thereof. The API may be Compound (a) or a pharmaceutically acceptable salt form thereof. The API may be Compound (a) in a solvated form, for example as a monohydrate. Preferably the API is Compound (a). Preferably the API is the (S)- enantiomer of Compound (a). Preferably the API is Compound (a) in anhydrous form. Preferably the API is Compound (a) in amorphous form. Preferably the API is Compound (a) or a pharmaceutically acceptable salt form thereof in amorphous form or dissolved state. Preferably the API is Compound (a) in amorphous form or dissolved state. Preferably the API is the (S)- enantiomer of Compound (a) in amorphous form. Preferably the API is the (S)- enantiomer of Compound (a) in anhydrous form.

In particular, the API used as starting material in the process to prepare a pharmaceutical formulation as described herein, is Compound (a), a solvated form, or a pharmaceutically acceptable salt form thereof; while the API in the final pharmaceutical formulation or solid dosage form is Compound (a) or a pharmaceutically acceptable salt form thereof in amorphous form or dissolved state.

In particular, the API used as starting material in the process to prepare a pharmaceutical formulation as described herein, is Compound (a) in a solvated form, or a pharmaceutically acceptable salt form thereof; while the API in the final pharmaceutical formulation or solid dosage form is Compound (a) or a pharmaceutically acceptable salt form thereof in amorphous form or dissolved state (i.e. molecular dispersion).

Compounds of formula (I) can be synthesized according to the procedures disclosed in WO 2016/180696, which is incorporated herein by reference in its entirety.

It will be appreciated that any of the above description relating to active pharmaceutical ingredients may apply to any embodiment of the pharmaceutical formulations, solid dosage forms, processes, uses, and methods of prevention, treatment, and viral inhibition described herein. For example, any reference to a dengue viral replication inhibitor may refer to a compound of formula (I), or a stereo- isomeric form, a pharmaceutically acceptable salt, solvate, cocrystal or polymorph thereof.

In a particular embodiment, the API in the pharmaceutical formulation as described herein is Compound (a), or a stereo- isomeric form, a pharmaceutically acceptable salt, solvate or polymorph thereof. In a particular embodiment, the API in the pharmaceutical formulation as described herein is Compound (a).

In a particular embodiment, the API in the pharmaceutical formulation as described herein is a dengue viral replication inhibitor in amorphous form or dissolved state. In a particular embodiment, the API in the pharmaceutical formulation as described herein is Compound (a) or a pharmaceutically acceptable salt form thereof, in amorphous form or dissolved state. In a particular embodiment, the API in the pharmaceutical formulation as described herein is Compound (a) in amorphous form or dissolved state.

### Solid dosage form

The invention also provides a solid dosage form comprising a pharmaceutical formulation as described herein.

The dosage form may be an oral dosage form (e.g. a capsule for oral administration). Alternatively, the dosage form may be an enteral dosage form. The solid dosage form may alternatively be a tablet.

The solid dosage form as described herein (e.g. a tablet) may contain from 0.1 mg to 3000 mg of the API, from 1 mg to 2000 mg of the API, from 5 mg to 1000 mg of the API, from 10 mg to 500 mg of the API, from 20 mg to 400 mg of the API, from 30 mg to 300 mg of the API, from 50 mg to 200 mg of the API, from 70 mg to 150 mg of the API, from 100 mg to 120 mg of the API or any particular amount or range comprised therein. The therapeutically effective amount for said API will vary as will the diseases, syndromes, conditions, and disorders being prevented or treated.

The solid dosage form as described herein (e.g. a tablet) may contain from 0.5 mg to 1000 mg of the API. In some embodiments the solid dosage form may comprise from 0.5 mg to 800 mg, for example from 1.0 mg to 600 mg, for example from 2.0 mg to 450 mg; preferably the API is 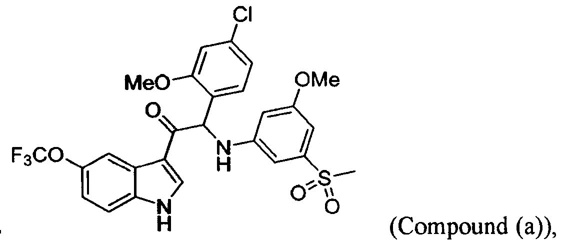 preferably the (S)- enantiomer of Compound (a). The solid dosage form may comprise at least 2 mg, at least 10 mg, at least 50 mg, at least 100 mg, at least 200 mg, at least 300 mg, at least 400 mg or at least 500 mg of Compound (a).

In a particular embodiment, the solid dosage form is a tablet comprising
a) an API; and
b1) methacrylic acid copolymer, or
b2) a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC), or a combination thereof;
wherein the API is a dengue viral replication inhibitor.

In a particular embodiment, the solid dosage form is a tablet comprising:
a) an API;
b1) methacrylic acid copolymer, or
b2) hydroxypropyl methylcellulose (HPMC); and
d) one or more pharmaceutically acceptable excipients selected from disintegrants, binders, diluents, lubricants, stabilizers, wetting agents, glidants, osmotic agents, colorants, plasticizers, and coatings;
wherein the API is a dengue viral replication inhibitor.

In a particular embodiment, the solid dosage form is a tablet comprising a pharmaceutical formulation of the present invention.

In an embodiment, the solid dosage form comprises a pharmaceutical formulation, wherein the formulation comprises at least 5 mg, at least 15 mg, at least 25 mg, at least 50 mg, at least 55 mg, at least 100 mg, at least 150 mg, at least 200 mg, at least 250 mg, at least 300 mg, at least 350 mg, at least 400 mg, at least 450 mg or at least 500 mg of the API; preferably the API is 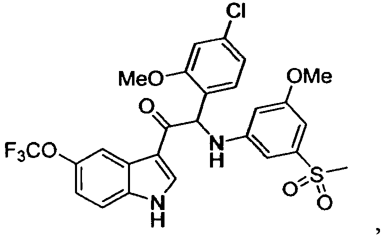 or a pharmaceutically acceptable salt form thereof.

For oral administration, a solid dosage form is in particular provided in the form of tablets containing at least 1.0 mg, at least 0.5 mg, at least 1 mg, at least 5 mg, at least 10 mg, at least 20 mg, at least 30 mg, at least 40 mg, at least 50 mg, at least 60 mg, at least 70 mg, at least 80 mg, at least 90 mg, at least 100 mg, at least 110 mg, at least 120 mg, at least 130 mg, at least 140 mg, at least 150 mg, at least 160 mg, at least 170 mg, at least 180 mg, at least 190 mg, at least 200 mg, at least 210 mg, at least 220 mg, at least 230 mg, at least 240 mg, at least 250 mg, at least 260 mg, at least 270 mg, at least 280 mg, at least 290 mg, at least 300 mg, at least 310 mg, at least 320 mg, at least 330 mg, at least 340 mg, at least 350 mg, at least 360 mg, at least 370 mg, at least 380 mg, at least 390 mg, at least 400 mg, at least 410 mg, at least 420 mg, at least 430 mg, at least 440 mg, at least 450 mg, at least 460 mg, at least 470 mg, at least 480 mg, at least 490 mg, and at least 500 mg at least 510 mg, at least 520 mg, at least 530 mg, at least 540 mg, at least 550 mg, at least 560 mg, at least 570 mg, at least 580 mg, at least 590 mg, at least 600 mg, at least 610 mg, at least 620 mg, at least 630 mg, at least 640 mg, at least 650 mg, at least 660 mg, at least 670 mg, at least 680 mg, at least 690 mg, at least 700 mg, at least 710 mg, at least 720 mg, at least 730 mg, at least 740 mg, at least 750 mg, at least 760 mg, at least 770 mg, at least 780 mg, at least 790 mg, at least 800 mg, at least 810 mg, at least 820 mg, at least 830 mg at least 840 mg, at least 850 mg, at least 860 mg, at least 870 mg, at least 880 mg, at least 890 mg at least 900 mg, at least 910 mg, at least 920 mg, at least 930 mg, at least 940 mg, at least 950 mg at least 960 mg, at least 970 mg, at least 980 mg, at least 990 mg, at least 1000 mg, at least 1100 mg at least 1200 mg, at least 1210 mg, at least 1220 mg, at least 1230 mg, at least 1240 mg, at least 1250 mg at least 1260 mg, at least 1270 mg, at least 1280 mg, at least 1290 mg, at least 1300 mg, at least 1410 mg at least 1320 mg, at least 1330 mg, at least 1340 mg, at least 1350 mg, at least 1360 mg, at least 1370 mg at least 1390 mg, at least 1400 mg of API or any value comprised in the aforementioned values. In particular the solid dosage form comprises from 15 mg to 500 mg of API.

Advantageously, the API or the solid dosage form may be administered in a single daily dose, or the total daily dosage may be administered in divided doses of two, three, four or five daily. The daily dose may be maintained unchanged throughout all days or some days of a treatment or prevention period. Said daily dose may change throughout the days of a treatment or prevention period such as it increases and/or decreases during the days of said treatment or prevention period. For instance, said daily dose may be unchanged for the first 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 days or more followed by a lower and/or a higher daily dose for the remaining days of the treatment or prevention period. Said remaining days of the treatment or prevention period can be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 days or more. The API or the solid dosage form may also be administered at least once every week, at least once every two weeks, at least once every three weeks, at least once every four weeks or a month, at least once every two months, at least once every three months, at least once every four months, at least once every five months, at least once every sixth months or at least once a year. The API or the solid dosage form may also be administered daily for 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 days or more followed by at least an administration at least once every week, at least once every two weeks, at least once every three weeks, at least once every four weeks or a month, at least once every two months, at least once every three months, at least once every four months, at least once every five months, at least once every sixth months or at least once a year.

In certain embodiments, the API or the solid dosage form may be administered at a first dose for a first duration (e.g., a loading phase) and at a second dose for a second duration (e.g., maintenance phase). The loading phase may include administration of any of the dosages described herein (e.g., from about 10 mg to about 1000 mg, from about 25 mg to about 800 mg, or from about 50 mg to about 400 mg). The first duration of administration in the loading phase may be for any of the time periods contemplated herein (e.g., from about 1 day to about 40 days, from about 3 days to about 20 days, or from about 5 days to about 10 days). The maintenance phase may include administration of any of the dosages described herein (e.g., from about 10 mg to about 1000 mg, from about 25 mg to about 800 mg, or from about 50 mg to about 400 mg). The second duration of administration in the maintenance phase may be for any of the periods contemplated herein (e.g., from about 1 day to about 60 days, from about 5 days to about 45 days, or from about 10 days to about 30 days).

Optimal dosages of the pharmaceutical formulation to be administered may be readily determined and will vary with the particular compound used, the mode of administration, the strength of the preparation, and the advancement of the disease, syndrome, condition or disorder. The above dosages are thus exemplary of the average case. There can be, of course, individual instances wherein higher or lower dosage ranges are merited, and such are within the scope of this invention.

The invention also provides a process for preparing a pharmaceutical formulation, as described herein. The process comprises the steps of:
a) dissolving the API in a solvent to form a solution;
b) mixing methacrylic acid copolymer or hydroxypropyl methylcellulose or a combination thereof with the solution formed in step a) thereby obtaining a mixture, and for example further stirring the mixture;
c) spray drying the mixture to obtain a solid dispersion;
d) optionally blending the solid dispersion with one or more pharmaceutically acceptable excipients;
to provide a pharmaceutical formulation as described herein.

As used herein the term "solid dispersion" means the dispersion of an API in a solid matrix where the matrix comprises a small molecule or a polymer or a combination thereof.

The pharmaceutical formulation according to the present invention comprises a solid dispersion wherein the matrix is a polymer and the polymer is selected from methacrylic acid copolymer, or a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose (HPMC), or a combination thereof. Preferably, the cellulose derivative is HPMC.

The invention also provides a process for preparing a solid dosage form described herein, the process comprising the steps of:
a) dissolving the API in a solvent to form a solution;
b) mixing methacrylic acid copolymer or a cellulose derivative such as methyl cellulose (MC), ethyl cellulose (EC), hydroxyethyl cellulose (HEC), hydroxypropyl cellulose (HPC), carboxymethyl cellulose (CMC), sodium carboxymethyl cellulose (NaCMC) or hydroxypropyl methylcellulose, or a combination thereof with the solution formed in step a) thereby obtaining a mixture, and optionally further stirring the mixture;
c) spray drying the mixture to obtain a solid dispersion;
d) optionally blending the solid dispersion with one or more pharmaceutically acceptable excipients; for example, the pharmaceutically acceptable excipient can be selected from the group comprising fillers, surfactants, disintegrants, glidants, lubricants and mixture thereof;
e) compressing the blend into a tablet;
to provide a solid dosage form as described herein.

In some embodiments, the process for preparing a solid dosage form described herein, comprises the steps of:
a) dissolving the API in a solvent to form a solution;
b) mixing the methacrylic acid copolymer or hydroxypropyl methylcellulose or a combination thereof with the solution formed in step a) thereby obtaining a mixture, and optionally further stirring the mixture;
c) spray drying the mixture to obtain a solid dispersion;
d) blending the solid dispersion with at least one filler, at least one surfactant, at least one disintegrant, at least one glidant and at least one lubricant;
e) granulating the blend;
f) blending the mixture obtained in step e) with at least one disintegrant, filler and at least one lubricant;
g) compressing the blend into a tablet;
to provide a solid dosage form as described herein.

In some embodiments, the solid dispersion may be obtained using hot melt extrusion. In some embodiments the step of granulating the blend is performed using a roller compactor or by slugging.

Another aspect of the present invention provides a packaged pharmaceutical formulation, wherein the pharmaceutical formulation is any formulation described herein (e.g., a tablet) sealed in a blister film, wherein the blister film comprises a formulation retaining layer configured to hold one or more pharmaceutical formulation (e.g., tablets) and a sealing layer configured to overlay the retaining layer to seal the pharmaceutical formulation (s) within the retaining layer, wherein the sealing layer comprises aluminium foil and a desiccant material. As used herein, the term "desiccant material" refers to any hygroscopic substance useful as a drying agent. Examples of desiccant materials include without limitation silica (e.g., silica gel), activated charcoal, calcium sulfate, calcium chloride, and zeolite materials.

In some embodiments, the retaining layer comprises one or more chambers, wherein each chamber is configured to hold one or more pharmaceutical formulations (such as any pharmaceutical formulation described herein (e.g., one or more tablets), and each chamber is sealed by the sealing layer. In some embodiments, the retaining layer comprises a clear or opaque material (e.g., a clear or opaque polyethylene material). In some embodiments, the sealing layer entirely overlaps the retaining layer and any chambers provided in the retaining layer.

Examples of commercially available blister films useful for the present invention include Dessiflex Plus and Dessiflex Ultra available from Amcor plc. In some embodiments, the packaged pharmaceutical formulation consists of 1 or more (e.g., 1, 2, 3, 4, 5, 6, 7, 8, 9, 10, 1 to 4, 2 to 10, or 1 to 10) tablets sealed in the blister film, wherein the blister film comprises one card. The stability and shelf life of the pharmaceutical formulations of the instant invention are improved using the blister film packaging of the instant invention, wherein the sealing layer comprises a desiccant material, as compared to packaging the pharmaceutical formulation in a blister film having a sealing layer that lacks a desiccant material (e.g., Aclar 400 blister film).

Another aspect of the present invention provides a kit comprising a packaged pharmaceutical formulation, such as any packaged pharmaceutical formulation described herein, and instructions for the administration of the packaged pharmaceutical formulation.

It will be appreciated that any of the above discussion relating to solid dosage forms and processes for their preparation may apply to any embodiments of solid dosage forms, processes prevention and treatments described herein.

### Methods of prevention or treatment or inhibition

The present invention also encompasses the pharmaceutical formulations and solid dosages described herein for use as a medicament. The present invention further encompasses a method for the prevention of a dengue viral infection or for the treatment of a dengue viral infection or for inhibiting viral replication of a Dengue virus in a biological in vitro sample or in a subject. The method comprises administering to the in vitro sample or the subject (in need thereof) an effective amount of the pharmaceutical formulations and solid dosages described herein. The in vitro sample or the subject are at risk of being infected by Dengue virus or infected by Dengue virus.

The pharmaceutical formulations described herein may be administered in any of the foregoing dosage forms and regimens or by means of those dosage forms and regimens established in the art whenever use of the pharmaceutical formulation is required for a subject in need thereof.

The pharmaceutical formulations and dosage forms of the present invention are useful in methods for treating, ameliorating and/or preventing a disease, a syndrome, a condition or a disorder in a subject in need thereof. Such methods comprise, consist of and/or consist essentially of administering to a subject, including an animal, a mammal, and a human in need of such treatment, amelioration and/or prevention, a therapeutically effective amount of a formulation or dosage form described herein. In embodiments in which the active pharmaceutical ingredient is a dengue viral replication inhibitor, the pharmaceutical formulations and dosage forms of the present invention are useful in methods for treating, ameliorating and/or preventing a disease, a syndrome, a condition that is affected by the inhibition of dengue viral replication.

One embodiment of the present invention is directed to a method of preventing a dengue viral infection in a subject in need thereof, including an animal, a mammal, and a human in need of such prevention, comprising administering to the subject a therapeutically effective amount of a pharmaceutical formulation or dosage form described herein.

One embodiment of the present invention is directed to a method of treating a dengue viral infection in a subject in need thereof, including an animal, a mammal, and a human in need of such treatment, comprising administering to the subject a therapeutically effective amount of a pharmaceutical formulation or dosage form described herein.

One embodiment of the present invention is directed to a method of inhibiting viral replication of a Dengue virus in a subject in need thereof, including an animal, a mammal, and a human in need of such treatment, comprising administering to the subject a therapeutically effective amount of a pharmaceutical formulation or dosage form described herein.

In certain embodiments, the blood plasma level of the API is at a level, e.g., for the duration of the treatment regimen (for treatment or for prevention), that is in the range of about 5 ng/ml to about 10,000 ng/ml, about 10 ng/ml to about 8,000 ng/ml, about 15 ng/ml to about 6,500 ng/ml, about 20 ng/ml to about 5,000 ng/ml, about 25 ng/ml to about 4,500 ng/ml, about 30 ng/ml to about 3,000 ng/ml, about 40 ng/ml to about 2,000 ng/ml, or about 50 ng/ml to about 1,000 ng/ml, or any single value or sub-range therein. In certain embodiments, the maximum blood plasma level of the API is up to about 10,000 ng/ml, up to about 8,000 ng/ml, up to about 6,500 ng/ml, up to about 4,500 ng/ml, up to about 3,000 ng/ml, up to about 2,000 ng/ml, up to about 1,000 ng/ml or any single value or sub-range therein. In certain embodiments, the minimum blood plasma level of the API, e.g., for the duration of the treatment regimen (for treatment or for prevention), is at least about 5 ng/ml, at least about 10 ng/ml, at least about 15 ng/ml, at least about 20 ng/ml, at least about 25 ng/ml, at least about 30 ng/ml, at least about 40 ng/ml, at least about 50 ng/ml, or any single value or sub-range therein. The blood plasma levels referred to here may be obtained with any of the doses and/or dosing regimens described herein.

In another embodiment of the present invention, the pharmaceutical formulations described herein may be employed in combination with one or more other medicinal agents, more particularly with other antiviral agents.

It will be appreciated that variations to the foregoing embodiments of the invention can be made while still falling within the scope of the invention. Each feature disclosed in this specification, unless stated otherwise, may be replaced by alternative features serving the same, equivalent or similar purpose. Thus, unless stated otherwise, each feature disclosed is one example only of a generic series of equivalent or similar features.

### Administration Methods

The compounds and pharmaceutically acceptable formulations described above can be administered to humans and other animals orally, rectally, intracisternally, intravaginally, intraperitoneally, topically (as by powders, ointments, or drops), buccally, as an oral or nasal spray, or the like, depending on the severity of the infection being prevented or treated.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofuryl alcohol, polyethylene glycols and fatty acid esters of sorbitan, and mixtures thereof. Besides inert diluents, the oral formulations can also include adjuvants such as wetting agents, emulsifying and suspending agents, sweetening, flavoring, and perfuming agents.

Formulations for rectal or vaginal administration are specifically suppositories which can be prepared by mixing the compounds described herein with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) diluents or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid formulations of a similar type may also be employed as diluents in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a formulation that they release the active ingredient(s) only, or preferentially, in a certain part of the intestinal tract, optionally, in a delayed manner. Examples of embedding formulations that can be used include polymeric substances and waxes. Solid formulations of a similar type may also be employed as diluents in soft and hard-filled gelatin capsules using such excipients as lactose or milk sugar as well as high molecular weight polyethylene glycols and the like
The active compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a formulation that they release the active ingredient(s) only, or preferentially, in a certain part of the intestinal tract, optionally, in a delayed manner. Examples of embedding formulations that can be used include polymeric substances and waxes.

Dosage forms for topical or transdermal administration of a compound described herein include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

The formulations described herein may be administered orally, by inhalation spray, topically, rectally, nasally, buccally, vaginally or via an implanted reservoir.

The pharmaceutical formulations described herein may be orally administered in any orally acceptable dosage form including, but not limited to, capsules, tablets, aqueous suspensions or solutions. In the case of tablets for oral use, carriers commonly used include, but are not limited to, lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical formulations described herein may be administered in the form of suppositories for rectal administration. These can be prepared by mixing the agent with a suitable non-irritating excipient which is solid at room temperature but liquid at rectal temperature and therefore will melt in the rectum to release the drug. Such materials include, but are not limited to, cocoa butter, beeswax and polyethylene glycols.

The pharmaceutical formulations described herein may also be administered topically, especially when the target of prevention and/or treatment includes areas or organs readily accessible by topical application, including diseases of the eye, the skin, or the lower intestinal tract. Suitable topical formulations are readily prepared for each of these areas or organs. Topical application for the lower intestinal tract can be effected in a rectal suppository formulation (see above) or in a suitable enema formulation. Topically-transdermal patches may also be used.

For topical applications, the pharmaceutical formulations may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical formulations can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2 octyldodecanol, benzyl alcohol and water.

The pharmaceutical formulations may also be administered by nasal aerosol or inhalation. Such formulations are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The compounds for use in the methods of the invention can be formulated in unit dosage form. The term "unit dosage form" refers to physically discrete units suitable as unitary dosage for subjects undergoing prevention or treatment, with each unit containing a predetermined quantity of active material calculated to produce the desired therapeutic effect, optionally in association with a suitable pharmaceutical carrier. The unit dosage form can be for a single daily dose or one of multiple daily doses (e.g., 1 to 4 or more times per day). When multiple daily doses are used, the unit dosage form can be the same or different for each dose.

All possible combinations of the above-indicated embodiments are considered to be embraced within the scope of this invention.

Reference is now made to the following examples, which illustrate the invention in a non-limiting fashion.

### GENERAL SYNTHETIC METHODS

Representative compounds for use in the present invention can be synthesized in accordance with the general synthetic methods described below and illustrated in the schemes and examples that follow. Since the schemes are an illustration, the invention should not be construed as being limited by the chemical reactions and conditions described in the schemes and examples. Compounds analogous to the target compounds of these examples can be made according to similar routes. The disclosed compounds are useful as pharmaceutical agents as described herein. The various starting materials used in the schemes and examples are commercially available or may be prepared by methods well within the skill of persons versed in the art.

### Compounds of Formula I.

The synthesis of compounds of general formula **I** can be performed as outlined in Scheme 1. 2-(4-Chloro-2-methoxyphenyl)acetic acid **(II)** can be converted to the corresponding 2-(4-chloro-2-methoxyphenyl)acetyl chloride **(III)** with a chlorination reagent like for example thionyl chloride. The Friedel-Crafts reaction of the acid chloride **III** with a substituted indole of general formula **IV** can be performed using a Lewis acid reagent like for example Et₂AlCl or TiCl₄ in a suitable solvent like for example CH₂Cl₂ or 1,2-dichloroethane, and under suitable reaction conditions that typically (but not exclusively) involve cooling, to provide the 3-acylated indole of general formula **V.** The introduction of an aniline moiety in alpha position to the carbonyl moiety of the compounds of general formula **V** can be accomplished by a reaction sequence that involves for example bromination of **V** with a reagent like for example phenyltrimethylammonium tribromide in a suitable solvent like for example THF (tetrahydrofuran), to provide the compounds of general formula **VI,** and subsequent reaction of the compounds of general formula **VI** with 3-methoxy-5-(methylsulfonyl)aniline **(VII)** in a suitable solvent like for example CH₃CN, and typically using a base like for example triethylamine (TEA) or N,N-Diisopropylethylamine (DIPEA), to provide the compounds of general formula **I** as racemic mixtures. Chiral separation of the compounds of general formula **I** can be performed by for example chiral chromatography to provide the Enantiomers **A** and **B** of general formula **I.**

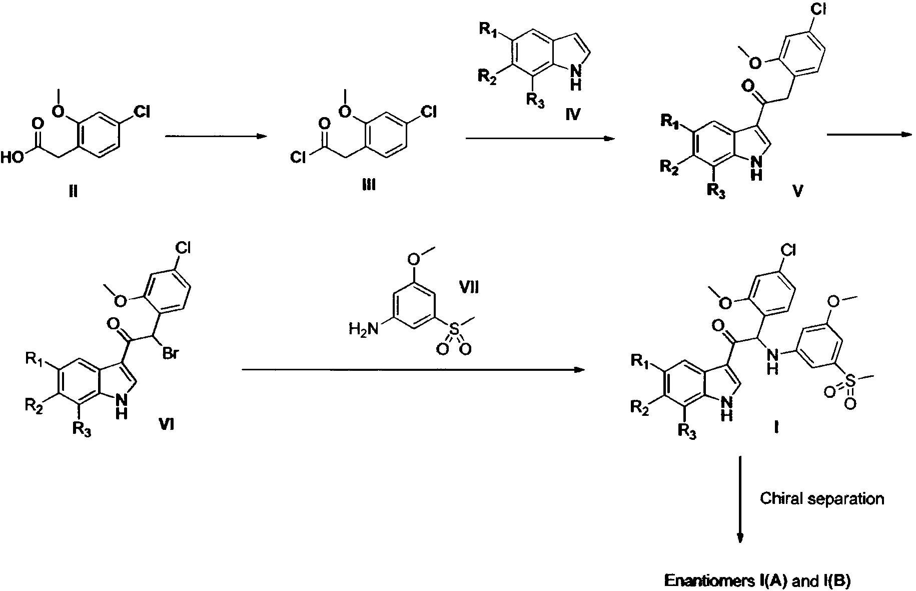

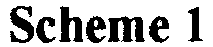

In some cases, the synthesis of the intermediate of general formula **V** via the Friedel-Crafts synthesis approach, benefits from the presence of a protecting group (PG) at the indole-N during the Friedel-Crafts reaction step, as outlined in Scheme 2. To this end, the substituted indole of general formula **IV** can be converted first to an N-protected intermediate of general formula **VIII,** such as for example an N-Tosylated intermediate of general formula **VIII** (PG = Ts), using a reagent like for example tosyl chloride, in the presence of a base like for example sodium hydride. The Friedel-Crafts reaction of the substituted indole of general formula **IV** with acid chloride **III** can be performed using a Lewis acid reagent like for example Et₂AlCl or TiCl₄ in a suitable solvent like for example CH₂Cl₂ or 1,2-dichloroethane, and under suitable reaction conditions that typically (but not exclusively) involve cooling, to provide the 3-acylated N-protected indole of general formula **IX.** Removal of the indole-N protecting group PG of the intermediate of general formula **IX** can be accomplished with a reagent like for example LiOH (for PG = Ts) in a solvent mixture like for example THF/water an at a suitable reaction temperature, to provide the 3-acylated indole of general formula **V.**

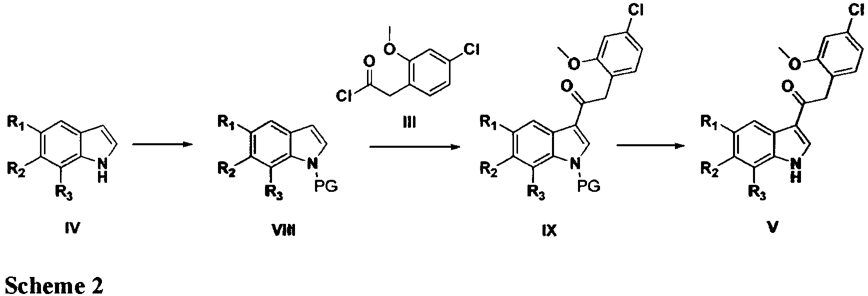

As an alternative approach, the intermediate of general formula **V** can also be prepared as outlined in Scheme 3: The *N*-*Boc*-protected substituted indole-3-carbaldehyde of general formula **X** can be converted to the corresponding Strecker-type of intermediate of general formula **XI** by reaction with morpholine in the presence of reagents like for example sodium cyanide and sodium bisulfite and in a suitable solvent like for example a mixture of water and a water-mixable organic solvent like for example dioxane. Alkylation of the compound of general formula **XI** with 4-chloro-2-methoxy-benzylchloride can be accomplished in the presence of a base like for example potassium hexamethyldisilazane and in a suitable solvent like for example dimethylformamide (DMF) to provide the compound of general formula **XII.** Submission of the compound of general formula **XII** to a suitable aqueous acidic hydrolytic condition like for example by treatment with an aqueous hydrochloric acid solution at elevated temperature, provides the intermediate of general formula **V.**

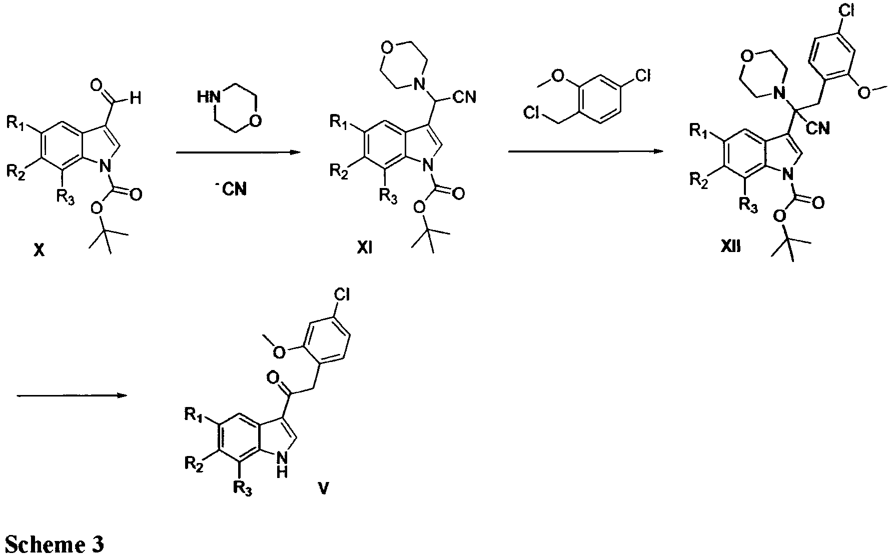

Compounds of Formula (I) can be synthesized according to the procedures disclosed in WO 2016/180696, which is incorporated herein by reference in its entirety.

### EXAMPLES

In the following examples compound (a), preferably the (+)-enantiomer of compound (a), is used as active pharmaceutical ingredient (API). Compound (a) was synthesized as described in WO 2016/180696, under Example 9. It was obtained as a white powder:

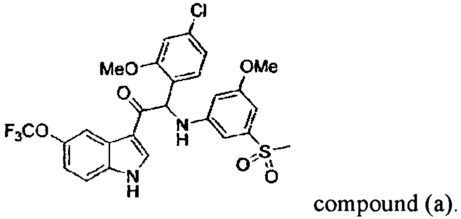

### Reagents

| **Chemical Name** | | **Company** |
|---|---|---|
| methacrylic acid - methyl methacrylate copolymer | Eudragit L100 | Evonik |
| Poly[methacrylic acid, ethyl acrylate] | Eudragit L100-55 | Evonik |
| hydroxypropyl methylcellulose E5 | HPMC E5 | Dow |
| Hydroxypropyl methylcellulose acetate succinate | HPMC-AS | Shin-Etsu |
| Hydroxypropyl methylcellulose acetate succinate | HPMC AS MG | Shin-Etsu |
| Copovidone | PVP VA 64 | BASF |
| Polyvinylpyrrolidone | PVP K30 | BASF |
| Croscarmellose sodium: | Ac-Di-Sol^{®} | DuPont Pharma |
| Microcrystalline cellulose: | Avicel^{®} PH102 | DuPont Pharma |
| Colloidal fumed silica | Aerosil^{®} 200 | Evonik |
| Magnesium Stearate | N/A | Avantor |
| Sodium lauryl sulfate | Kolliphor^{®} SLS Fine | BASF |
| Mannitol | N/A | Roquette |
| Vitamine E TPGS | N/A | Isochem |

### Example 1: Solid dispersion screen

Solid dispersion formulations were prepared in a 96-well plate by a solvent evaporation method (transfer 100µL of liquid containing the required amount of API and additives to each well, transfer well plate to a vacuum oven at 70°C and < 2mbar pressure for one hour, cool down to room temperature). API (Compound (a) in amorphous form) and excipients were both dissolved in a mixture of dichloromethane and methanol (50/50, v/v). Mixtures were prepared using an automated liquid handling workstation (Hamilton Microlab STAR plus). After dispensing, amorphous API-polymer films were generated by rapid evaporation of the organic solvent. This was achieved by evaporation under reduced pressure for one hour using a vacuum oven set at 70°C and 200 mbar. The solid dispersions prepared are indicated in Table A. The resulting films (12 replicates of each formulation per screen), each containing approximately 100 µg of API, were cooled down and kept at room temperature, for one day for the first screen, and for 3 days for the second screen, before starting the dissolution assay. Films were prepared with a constant percentage of API, 33 wt%, while the sum of the excipients was 67 wt%. As reference, a film containing only API was included.

**Table A. tested solid dispersions**

| **Solid Dispersion** | **Weight ratio of API / excipient 1 / excipient 2* /excipient 3*** | **API load** |
|---|---|---|
| Dispersion 1 | API/PVP K30/Eudragit L100 (1/0.6/1.4) | 100 µg |
| Dispersion 2 | API/PVP K30/Eudragit L100/SLS (1/0.6/1.37/0.04) | 100 µg |
| Dispersion 3 | API/PVP K30/HPMC E5 (1/0.6/1.4) | 100 µg |
| Dispersion 4 | API/PVP K30/Vit E TPGS (1/1.96/0.04) | 100 µg |
| Dispersion 5 | API/PVP K30 (1/2) | 100 µg |
| Dispersion 6 | API/PVP K30/SLS (1/1.96/0.04) | 100 µg |
| Dispersion 7 | API/PVP K30/HPMC AS LG (1/0.6/1.4) | 100 µg |
| Reference | API | 100 µg |

| | | |
|---|---|---|
| ** if present* | | |

A crystallinity assessment was performed by polarized light microscopy after film casting, the day the dissolution study started and after one, two and four weeks of stability at 40°C/75% humidity. No crystalline material could be detected after film casting, the day the dissolution study started and after one, two and four weeks of stability at 40°C/75% relative humidity.

*In vitro,* 2-phase (SGF/FaSSIF) miniaturized dissolution was performed, where the amount of dissolved API was monitored as a function of time. Before starting the dissolution assay, the films were stored at room temperature, for one day for the first screen and for 3 days for the second screen. By doing so, most of the residual solvent was evaporated. Actual dissolution experiments were performed in 96 1 mL glass vials using the Hamilton STAR plus liquid handling platform for both sampling and sample preparation. Before addition to the films, the media was preheated to 37°C. 300 µL of preheated SGF (37°C, pH 1.3) was then added to the solid dispersions. After 15 minutes of incubation in SGF, 600 µL of preheated concentrated FaSSIF (37°C, pH 10.5) was added to the samples. The addition of this concentrated FaSSIF to SGF results in a medium with a similar composition to the typical FaSSIF medium used in 1-phase dissolution studies. At predetermined time intervals (11, 24, 34, 49, 79, 139 minutes), aliquots were withdrawn from the dissolution media and filtered through a 0.45 µm GHP membrane filter. Subsequently, the filtered solutions were quantitatively diluted with N-methyl pyrrolidone (NMP) to prevent possible precipitation. The amount of dissolved API was determined by UPLC/UV-Vis analysis. Experiments were performed in duplicate for each formulation per screen.

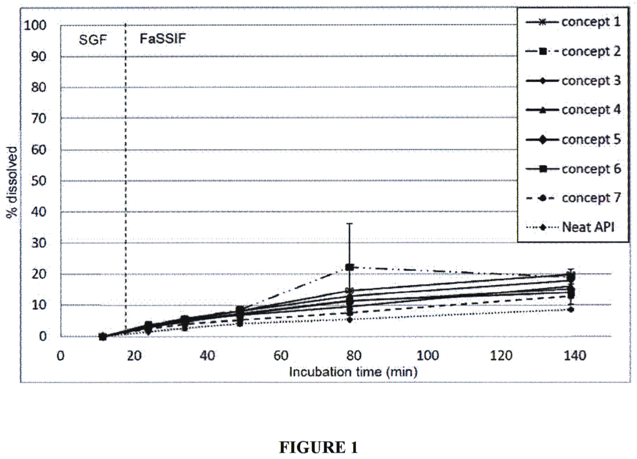

Figure 1 shows the dissolution profiles of solid dispersions 1 to 7 (corresponding to concepts 1 to 7 in Figure 1) in SGF-FaSSIF. A final release of approximately 9% of the total amount of API present in the films was measured for the neat amorphous API reference. All other tested solid dispersions show improved dissolution profiles compared to the reference. In the gastric phase, the API did not dissolve in any solid dispersion. For Solid dispersion 1, a final release of 20% of API was reached.

A second solubility screen was performed as described above for solid dispersions 8 to 12 and their compositions listed in Table B. These solid dispersions were prepared as described above.

**Table B. tested solid dispersions**

| **Solid Dispersion** | **Weight ratio of API / excipient 1 / excipient 2* /excipient 3*** | **API load** |
|---|---|---|
| Dispersion 8 | API/Eudragit L100 (1/2) | 100 µg |
| Dispersion 9 | API/PVP K30/Eudragit L100 (1/0.6/1.4)^{a} | 100 µg |
| Dispersion 10 | API/PVP K30/Eudragit L100/SLS (1/0.6/1.37/0.04)^{a} | 100 µg |
| Dispersion 11 | API/HPMC E5 (1/2) | 100 µg |
| Dispersion 12 | API/PVP K30/HPMC E5 (1/0.6/1.4)^{a} | 100 µg |

| | | |
|---|---|---|
| ** if present* *^{a}: the solid dispersion also has been tested in the first screen* | | |

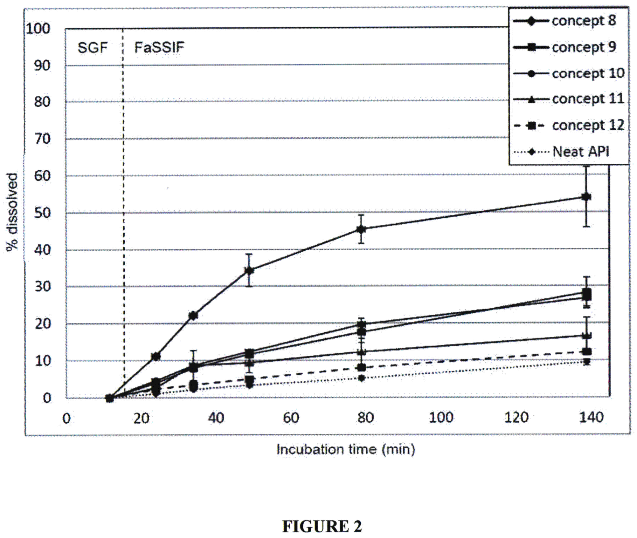

Figure 2 shows the dissolution profiles of solid dispersions 8 to 12 (corresponding to concepts 8 to 12 in Figure 2) in SGF-FaSSIF. A final release of approximately 9% of the total amount of API present in the films was measured for the neat amorphous API reference. For Dispersion 8, a final release of 54% API is reached, while for dispersion 9, a final release of 28% of API was reached.

### Example 2: Solubility screening

To screen appropriate solvent for spray drying, approximate solubility of starting material was estimated in different organic solvents at ambient temperature. Each solvent was added in increments of 50 µL or 100 µL into a 2 mL glass vial containing about 5 mg of each sample (API, Eudragit L100 and HPMC E5), until the solids were dissolved or a total volume of 1 mL was reached. Results are summarized in Table below.

**Table 1 Solubility screening of API, Eudragit L-100 and HPMC E5; S (solubility) in mg/mL**

| **Solvent\Polymer** | **API** | **Eudragit L100** | **HPMC E5** |
|---|---|---|---|
| Acetone/EtOH (1/1, v/v) | S>100 | S>100 | S<3.3 |
| Acetone/EtOH (2/1, v/v) | S>100 | S>100 | S<3.3 |
| Acetone/H2O (9/1, v/v) | S>100 | S>100 | S<3.3 |
| EtOH/H2O (9/1, v/v) | 10<S<12.5 | S>100 | S<3.3*¹ (co-solvent system directly) |
| EtOH/H2O (9:1, v/v) | N/A | N/A | S~25*² (water first) |
| Acetone/MeOH (2/1, v/v) | S>100 | N/A | S<3.3 |
| MeOH/DCM (1/1, v/v) | S>100 | 150<S<300 | 5~50* |
| MeOH/DCM (3/1, v/v) | N/A | N/A | 3.3~12.5* |
| MeOH | 42 | S>300 | N/A |

| | | | |
|---|---|---|---|
| ** Clear solution with very few particles, probably due to the intrinsic property of HPMC E5 itself.* **¹ HPMC E5 was added into mixture of organic solvent systems directly.* **² HPMC E5 was dissolved in water at first, then organic solvents were added* | | | |

Equilibrium solubility in MeOH and MeOH/dichloromethane (DCM) (1/1, v/v) was determined by UPLC, and was 42 mg/mL and 317 mg/mL, respectively.

### Example 3: Spray drying

Based on the solvent selection and polymer screening results mentioned in the above sections, three polymers including Eudragit L100, HPMC AS and HPMC E5 were selected as carrier materials, Acetone/EtOH (2:1, v/v) (for Eudragit L100) and MeOH/DCM (1:1, v/v) (for HPMC E5 and HPMC AS) were respectively selected as solvent system for spray drying.

Sufficient amounts of different polymers were weighed into suitable glass bottles respectively, added with sufficient amount of selected solvents to dissolve. About 25 g of Compound (a) (in amorphous state) was then weighed and dissolved into each of the above solutions respectively, to obtain clear solutions in Acetone/EtOH (2:1, v/v) at API concentration of 14 mg/mL for Eudragit L100 and clear solutions in MeOH/DCM (1:1, v/v) at API concentration of 10 mg/mL for HPMC E5 and HPMC AS, respectively. About 35 g of Compound (a) was also weighed and dissolved into acetone to obtain clear solution at API concentration of 50 mg/mL for making amorphous API.
Solid dispersion 1 (SD1): API + Eudragit L100 (1:2 w/w).
Solid dispersion 2 (SD2): API + HPMC E5 (1:2 w/w).
Solid dispersion 3 (SD2): API + HPMC AS (1:2 w/w).
Spray drying apparatus: Buchi B-290.

**Table 2 Spray drying conditions and results**

| **System** | **API** | **SD1** | **SD2** | **SD3** |
|---|---|---|---|---|
| **Drug load (wt%)** | 100% | 33.3% | 33.3% | 33.3% |
| **Mass composition (g)** | 35 g | 25g:50g | 25g:50g | 25g:50g |
| **API conc. in SDD solution (mg/mL)** | 50 | 14 | 10 | 10 |
| **Nozzle size (mm)** | 0.7 | 0.7 | 0.7 | 0.7 |
| **Solvent** | Acetone | Acetone/EtOH (2/1, v/v) | MeOH/DCM (1:1, v/v) | MeOH/DCM (2:8, w/w) |
| **Inlet temperature (°C)** | 90 | 100 | 85 | 75 |
| **Outlet temperature (°C)** | 59 | 67 | 55 | 53 |
| **Nozzle air flow (m³/mm)** | 40 | 50 | 40 | 40 |
| **Pump speed** | 50 | 40 | 50 | 50 |
| **Cyclone type** | High efficient | High efficient | High efficient | Standard |
| **Condenser temp. (°C)** | -21 | -20 | -20 | -20 |
| **Yield (%)** | 86.4 | 73.8 | 80.4 | 86.5 |

The spray dried dispersions (SDD) and the amorphous API spray dried power were further dried under vacuum at temperature below Tg of the mixture.

### Example 4: Solid Dispersion (SD) stability:

The obtained SD1, SD2 and SD3 powders, (i.e. Eudragit L100 based ASD1, HPMC E5 based ASD2, HPMC AS based ASD3), after final post-drying were directly weighed in vials and set up for physical and chemical stability.

Suitable amount of each SD product was weighed into 40 mL glass vials (i.e. 12 mg for chemical stability and 50 mg for physical stability). Then the sample vials were sealed with gaskets and caps closely and wrapped with aluminum foil and stored in stability chamber of 25°C/60%RH (closed) and 40°C/75%RH (closed) for different time points (2 weeks, 1 month, 2 months, 3 months and 6 months). Triplicate samples were set up for chemical stability under each condition for each time point and single for physical stability. Compound (a), Eudragit L100 and HPMC E5 were also weighed into 40 mL glass vials and set up at each condition and each time point for API and excipient control. Assay and impurity were tested for each condition at each time point for chemical stability and XRPD, PLM and mDSC were tested for physical stability.

Stability of SD1 as produced in Example 3 was tested by placing SD1 powder in glass bottles under stressed conditions as described in the first column of Table 3.

**Table 3 Physical and chemical stability of SD1**

| **Storage Condition** | **Time in months** | **PLM** | **XRPD** | **Appearance** | **No.** | **Total impurity (%)** | **Purity (%)** |
|---|---|---|---|---|---|---|---|
| Initial | N/A | N | Am | P1 | 1 | 0.93 | 99.07 |
| | | | | | 2 | 0.93 | 99.07 |
| 25 °C 60%RH (closed) | 1 M | N | Am | P1 | 1 | 0.88 | 99.12 |
| | | | | | 2 | 0.88 | 99.12 |
| | 2 M | N | Am | P1 | 1 | 0.91 | 99.09 |
| | | | | | 2 | 0.91 | 99.09 |
| | 3 M | N | Am | P1 | 1 | 0.93 | 99.06 |
| | | | | | 2 | 0.93 | 99.05 |
| | 6 M | N | Am | P1 | 1 | 0.91 | 99.09 |
| | | | | | 2 | 0.91 | 99.09 |
| 40 °C 75%RH (closed) | 1 M | N | Am | P1 | 1 | 0.92 | 99.08 |
| | | | | | 2 | 0.92 | 99.08 |
| | 2 M | N | Am | P2 | 1 | 0.97 | 99.03 |
| | | | | | 2 | 0.99 | 99.01 |
| | 3 M | N | Am | P2 | 1 | 1.01 | 98.99 |
| | | | | | 2 | 1.01 | 98.99 |
| | 6 M | N | Am | P2 | 1 | 0.95 | 99.05 |
| | | | | | 2 | 0.98 | 99.02 |

| | | | | | | | |
|---|---|---|---|---|---|---|---|
| *"RH": Relative Humidity; "M": Months; "N": non-birefringent; "Am": amorphous;* *"P1": white fluffy powder; "P2": white fluffy powder, and some powder could attach to the bottom and* wall of the *vial; "PLM": Polarized Light Microscope.* | | | | | | | |

No obvious chemical and physical changes were observed after 6 months of storage in closed glass bottles at 25 °C and 60% relative humidity. However, an increase of total impurity was observed after 6 months of storage in closed glass bottles at 40 °C and 75% relative humidity, compared to initial total impurity.

### Example 5: Solid Dispersion 2 (SD2) stability:

Stability of SD2 as produced in Example 3 was tested by placing SD2 powder in glass bottles under stressed conditions as described in the first column of Table 4.

**Table 4 Physical and chemical stability of SD2.**

| **Storage Condition** | **Time Point** | **PLM** | **XRPD** | **Tg (°C) / mDSC** | **Appearance** | **No.** | **Total impurity (%)** | **Purity (%)** |
|---|---|---|---|---|---|---|---|---|
| Initial | N/A | N | Am | 121.9 | P3 | 1 | 0.87 | 99.13 |
| | | | | | | 2 | 0.89 | 99.11 |
| 25°C/ 60% RH (closed) | 1 M | N | Am | 118.5 | P3 | 1 | 0.83 | 99.17 |
| | | | | | | 2 | 0.83 | 99.17 |
| | 2 M | N | Am | 122.6 | P3 | 1 | 0.86 | 99.14 |
| | | | | | | 2 | 0.87 | 99.13 |
| | 3 M | N | Am | 122.6 | P3 | 1 | 0.86 | 99.14 |
| | | | | | | 2 | 0.87 | 99.13 |
| | 6 M | N | Am | 122.6 | P3 | 1 | 0.83 | 99.18 |
| | | | | | | 2 | 0.82 | 99.19 |
| 40°C/ 75% RH (closed) | 1 M | N | Am | 121.4 | P3 | 1 | 0.83 | 99.16 |
| | | | | | | 2 | 0.84 | 99.16 |
| | 2 M | N | Am | 122.9 | P3 | 1 | 0.87 | 99.13 |
| | | | | | | 2 | 0.87 | 99.13 |
| | 3 M | N | Am | 116.7 | P3 | 1 | 0.88 | 99.12 |
| | | | | | | 2 | 0.88 | 99.12 |
| | 6 M | N | Am | 116.7 | P3 | 1 | 0.84 | 99.16 |
| | | | | | | 2 | 0.84 | 99.16 |

| | | | | | | | | |
|---|---|---|---|---|---|---|---|---|
| *"RH": Relative Humidity; "M": Months; "N": non-birefringent; "Am": amorphous;* *"P3": white powder, and some powder could attach to the bottom and wall of the vial;* *"PLM": Polarized Light Microscope.* | | | | | | | | |

Compared to initial, no obvious chemical and physical changes were observed after 6 months of storage in closed glass bottles at 25 °C/60% relative humidity and at 40 °C/ 75% relative humidity.

### Example 5A: Solid Dispersion 3 (SD3) stability:

Stability of SD3 as produced in Example 3 was tested by placing SD3 powder in glass bottles under stressed conditions as described in the first column of Table 4A.

**Table 4A Physical and chemical stability of SD3.**

| **Storage Condition** | **Time Point** | **PLM** | **XRPD** | **Tg (°C) / mDSC** | **Appearance** | **No.** | **Total impurity** (%) | **Purity (%)** |
|---|---|---|---|---|---|---|---|---|
| Initial | N/A | N | Am | 109.5 | P3 | 1 | 0.08 | 99.92 |
| | | | | | | 2 | 0.08 | 99.92 |
| 25°C/ 60% RH (closed) | 2 W | N | Am | 109.2 | P3 | 1 | 0.10 | 99.91 |
| | | | | | | 2 | 0.10 | 99.90 |
| | 1 M | N | Am | 109.8 | P3 | 1 | 0.13 | 99.87 |
| | | | | | | 2 | 0.13 | 99.87 |
| | 3 M | N | Am | 109.2 | P3 | 1 | 0.16 | 99.84 |
| | | | | | | 2 | 0.16 | 99.84 |
| 40°C/ 75% RH (closed) | 2 W | N | Am | 109.4 | P3 | 1 | 0.13 | 99.88 |
| | | | | | | 2 | 0.10 | 99.89 |
| | 1 M | N | Am | 109.8 | P3 | 1 | 0.15 | 99.84 |
| | | | | | | 2 | 0.17 | 99.83 |
| | 3 M | N/A | Am | 109.0 | P3 | 1 | 0.20 | 99.80 |
| | | | | | | 2 | 0.19 | 99.81 |

| | | | | | | | | |
|---|---|---|---|---|---|---|---|---|
| *"RH": Relative Humidity; "W": Weeks; "M": Months; "N": non-birefringent; "Am": amorphous; "P3": white powder, and some powder could attach to the bottom and wall of the vial; "PIM": Polarized Light Microscope.* | | | | | | | | |

Compared to initial, the total impurities increased during storage in closed glass bottles particularly at 40 °C and 75% relative humidity (from 0.08% at T0 to ~0.2% at 3 months).

### Example 5B: HPMC E5 Solid dispersion dissolution test

Four solid dispersions comprising different API/HPMC E5 weight ratio (listed below) were prepared as described in Example 3 above.
Solid dispersion 2a (SD2a): API + HPMC E5 (1:1 w/w)
Solid dispersion 2b (SD2b): API + HPMC E5 (1:2 w/w)
Solid dispersion 2c (SD2c): API + HPMC E5 (1:3 w/w)
Solid dispersion 2d (SD2d): API + HPMC E5 (1:4 w/w)
The prepared solid dispersions were tested in physiology-based dissolution tests (PBDT) that utilizes a simulated intestinal fluid as medium. SD2a, SD2b, SD2c and SD2d were tested in PBDT in fasted condition. SD2a, SD2b and SD2d were tested in PBDT in fed condition. The details of the PBDT are provided in Tables 4B and 4B' respectively for fasted and fed conditions.

**Table 4B: PBDT to determine dissolution in fasted condition.**

| **Parameter** | **PB method** |
|---|---|
| Dissolution apparatus | Paddle (USP type 2, Ph. Eur., JP) |
| Dissolution medium temp. | 37.0 ± 0.5 °C |
| Dissolution medium volume | 900 mL |
| Dissolution medium | **Phase 1 (15 minutes):** 300 mL of SGF* pH1.3 |
| | **Phase 2 (2 hours):** 900 mL of FaSSIF pH 6.5, 600 mL of FaSSIF** (preheated at 37 °C) was added to the 300mL Phase 1 solution. The total volume of FaSSIF pH 6.5 is 900mL |
| Paddle rotation speed | 75 rpm |
| Sample filter | Whatman Spartan 0.2 µm RC (regenerated cellulose) membrane 30-mm diameter filter, or equivalent. |
| Analytical finish | HPLC with UV detection at 274 nm |

| | |
|---|---|
| *Simulated Gastric Fluid; **Fasting State Simulated Intestinal Fluid | |

**Table 4B': PBDT to determine dissolution in fed condition.**

| **Parameter** | **PB method** |
|---|---|
| Dissolution apparatus | Paddle (USP type 2, Ph. Eur., JP) |
| Dissolution medium temp. | 37.0 ± 0.5 °C |
| Dissolution medium volume | 900 mL |
| Dissolution medium | **Phase 1 (60 minutes):** 300 mL of phosphate buffer pH 4.9 + 2 g/L NaCl |
| | **Phase 2 (3 hours):** 900 mL of FeSSIF pH 5.0, 600 mL of FeSSIF** (preheated at 37 °C) was added to the 300mL Phase 1 solution. The total volume of FeSSIF pH 5.0 is 900mL |
| Paddle rotation speed | 75 rpm |
| Sample filter | Whatman Spartan 0.2 µm RC (regenerated cellulose) membrane 30-mm diameter filter, or equivalent. |
| Analytical finish | HPLC with UV detection at 274 nm |

| | |
|---|---|
| **Fed State Simulated Intestinal Fluid | |

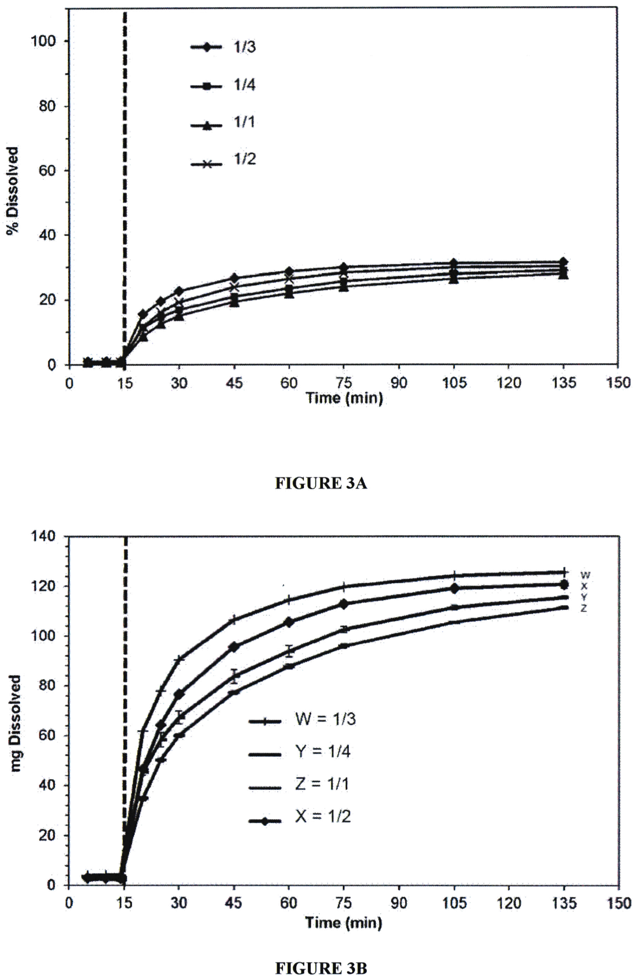

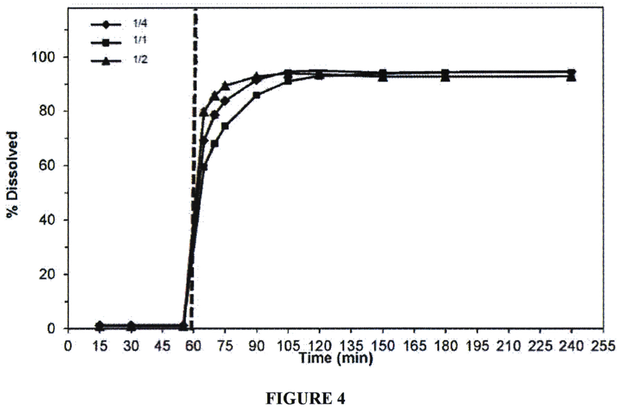

Figure 3A shows the dissolution percentage of the tested SD powders and Figure 3B shows the dissolution in mg of the same SD powders. SD2b and SD2c exhibit an improved dissolution profile compared to SD2a and SD2d. The improved dissolution profile was also observed for SD2b in PBDT fed condition as can be seen in Figure 4.

In addition, API and polymer dissolution of SD2a, SD2b, SD2c and SD2d was evaluated in SGF-FaSSIF by adding 50 mg of each SD to 150 mL pre-heated SGF pH 1.3. After 15 min, 280 mL pre-heated 1.5x concentrated FaSSIF pH 10.3 was added to the SGF pH 1.3, resulting in FaSSIF pH 6.5. The samples were stirred at 250 rpm in an incubator at 37°C. Time-dependent analysis were performed by taking 5 mL aliquots after 5' - 14' - 20' - 25' - 30' - 45' - 60' - 75' - 105' - 135'. The undissolved API was separated from the solution by filtration with a Millex LCR 25 mm filter with 0.45 µm PTFE (3 mL was discarded and 2 mL was used for the concentration measurements). The solutions were 2-fold dilution in ACN/water 50/50 v/v to avoid precipitation. The API concentration in solution was determined using UPLC-UV. The HPMC E5 concentration in solution was determined using UPLC-RI.

### Example 6: 10 mg SD1 tablets stability:

Tablets (10 mg strength) comprising SD1 were prepared. The composition of the tablets is shown in Table 5.

**Table 5: composition of SD1 Tablet (10 mg strength)**

| **Function** | **Component** | **wt%** | **mg/tablet** |
|---|---|---|---|
| SDD Powder | SD1 powder comprising API | 30.0% | 30 |
| Filler | Mannitol | 17.0% | 17 |
| Filler/Binder | Avicel PH102 | 45.0% | 45 |
| Surfactant | SLS | 1.0% | 1 |
| Disintegrant | Croscarmellose Sodium (Ac-Di-Sol) | 5.0% | 5 |
| Glidant | Colloidal Silicon dioxide (Aerosil 200) | 1.0% | 1 |
| Lubricant | Magnesium Stearate | 1.0% | 1 |

Stability of the API, SD1 powder as well as the tablets (10 mg strength) comprising SD1 was tested by placing the tablets in High-density polyethylene (HDPE) bottles under stressed conditions as described in column 2 of Table 6. The results are shown in Table 6.

**Table 6 Physical and chemical stability of 10 mg tablets prepared with Solid Dispersion 1.**

| **Sample** | **Storage Condition** | **Time Point** | **XRPD** | **Appearance** | **No.** | **Total impurity** (%) | **Purity (%)** |
|---|---|---|---|---|---|---|---|
| **API** | 40°C/ 75% RH (closed) | 2 W | N/A | T1 | 1 | 1.05 | 98.95 |
| | | 1M | N/A | T1 | 1 | 1.04 | 98.96 |
| | | 2M | N/A | T1 | 1 | 1.06 | 98.94 |
| | | 3 M | N/A | T1 | 1 | 1.05 | 98.95 |
| | 40°C/ 75% RH (open) | 2W | N/A | T1 | 1 | 1.05 | 98.95 |
| | | 1M | N/A | T1 | 1 | 1.06 | 98.94 |
| | | 2M | N/A | T1 | 1 | 1.04 | 98.96 |
| | | 3 M | N/A | T1 | 1 | 1.04 | 98.96 |
| **SD1 powder** | N/A | Initial | Am | T1 | 1 | 1.15 | 98.85 |
| | 40°C/ 75% RH (closed) | 2 W | Am | T1 | 1 | 1.26 | 98.75 |
| | | 1 M | Am | T1 | 1 | 1.33 | 98.67 |
| | | 2M | Am | T1 | 1 | 1.35 | 98.65 |
| | | 3 M | Am | T1 | 1 | 1.30 | 98.70 |
| | 40°C/ 75% RH (open) | 2 W | Am | T1 | 1 | 1.16 | 98.84 |
| | | 1 M | Am | T1 | 1 | 1.16 | 98.84 |
| | | 2 M | Am | T1 | 1 | 1.12 | 98.88 |
| | | 3 M | Am | T1 | 1 | 1.14 | 98.86 |
| **Tablet** | N/A | Initial | Am | T2 | 1 | 1.21 | 98.80 |
| | | | | | 2 | 1.22 | 98.78 |
| | 40°C/ 75% RH (closed) | 2 W | Am | T2 | 1 | 1.24 | 98.76 |
| | | | | | 2 | 1.23 | 98.77 |
| | | 1 M | Am | T2 | 1 | 1.25 | 98.75 |
| | | | | | 2 | 1.26 | 98.74 |
| | | 2M | Am | T2 | 1 | 1.28 | 98.72 |
| | | | | | 2 | 1.27 | 98.73 |
| | | 3 M | Am | T2 | 1 | 1.28 | 98.72 |
| | | | | | 2 | 1.29 | 98.71 |
| | 40°C/ 75% RH (open) | 2W | Am | T2 | 1 | 1.16 | 98.84 |
| | | | | | 2 | 1.16 | 98.84 |
| | | 1M | Am | T2 | 1 | 1.15 | 98.85 |
| | | | | | 2 | 1.16 | 98.84 |
| | | 2M | Am | T2 | 1 | 1.13 | 98.87 |
| | | | | | 2 | 1.13 | 98.87 |
| | | 3 M | Am | T2 | 1 | 1.13 | 98.87 |
| | | | | | 2 | 1.12 | 98.88 |

| | | | | | | | |
|---|---|---|---|---|---|---|---|
| ***"RH":** Relative humidity* ***"W":** week(s)* ***"M":** Month(s)* ***"Am":** amorphous;* ***"T1":** white powder;* ***"T2":** white or off white round tablet* | | | | | | | |

### Example 7: 10 mg SD2 tablets stability:

Tablets (10 mg strength) comprising SD2 were prepared. The composition of the tablets is shown in Table 7.

**Table 7: composition of SD2 Tablet (10 mg strength)**

| **Function** | **Component** | **wt%** | **mg/tablet** |
|---|---|---|---|
| SDD Powder | SD2 powder comprising API | 30.0% | 30 |
| Filler | Mannitol | 17.0% | 17 |
| Filler/Binder | Avicel PH102 | 45.0% | 45 |
| Surfactant | SLS | 1.0% | 1 |
| Disintegrant | CroscarmelloseSodium (Ac-Di-Sol) | 5.0% | 5 |
| Glidant | Colloidal Silicondioxide (Aerosil 200) | 1.0% | 1 |
| Lubricant | Magnesium Stearate | 1.0% | 1 |

Stability of the API, SD2 as well as the tablet (10 mg strength) comprising SD2 was tested by placing the tablets in HDPE bottles under stressed conditions as described in column 2 of Table 7. The results are shown in Table 7.

**Table 8 Physical and chemical stability of 10 mg tablets prepared with Solid Dispersion 2.**

| **Tablet** | **Storage Condition** | **Time Point** | **XRPD** | **Appearance** | **No.** | **Total impurity (%)** | **Purity (%)** |
|---|---|---|---|---|---|---|---|
| **API** | 40°C/ 75% RH (closed) | 2W | N/A | T1 | 1 | 1.05 | 98.95 |
| | | 1M | N/A | T1 | 1 | 1.04 | 98.96 |
| | | 2M | N/A | T1 | 1 | 1.05 | 98.94 |
| | | 3 M | N/A | T1 | 1 | 1.05 | 98.95 |
| | 40°C/ 75% RH (open) | 2W | N/A | T1 | 1 | 1.06 | 98.95 |
| | | 1M | N/A | T1 | 1 | 1.04 | 98.94 |
| | | 2M | N/A | T1 | 1 | 1.04 | 98.96 |
| | | 3 M | N/A | T1 | 1 | 1.05 | 98.96 |
| **SD-2 powder** | N/A | Initial | Am | T1 | 1 | 1.03 | 98.85 |
| | 40°C/ 75% RH (closed) | 2W | Am | T1 | 1 | 1.05 | 98.75 |
| | | 1M | Am | T1 | 1 | 1.07 | 98.67 |
| | | 2M | Am | T1 | 1 | 1.09 | 98.65 |
| | | 3M | Am | T1 | 1 | 1.08 | 98.70 |
| | 40°C/ 75% RH (open) | 2W | Am | T1 | 1 | 1.08 | 98.84 |
| | | 1M | Am | T1 | 1 | 1.10 | 98.84 |
| | | 2M | Am | T1 | 1 | 1.08 | 98.88 |
| | | 3 M | Am | T1 | 1 | 1.07 | 98.86 |
| **Tablet** | N/A | Initial | Am | T2 | 1 | 1.04 | 98.80 |
| | | | | | 2 | 1.04 | 98.78 |
| | 40°C/ 75% RH (closed) | 2W | Am | T2 | 1 | 1.04 | 98.76 |
| | | | | | 2 | 1.04 | 98.77 |
| | | 1M | Am | T2 | 1 | 1.05 | 98.75 |
| | | | | | 2 | 1.06 | 98.74 |
| | | 2M | Am | T2 | 1 | 1.07 | 98.72 |
| | | | | | 2 | 1.07 | 98.73 |
| | | 3 M | Am | T2 | 1 | 1.08 | 98.72 |
| | | | | | 2 | 1.07 | 98.71 |
| | 40°C/ 75% RH (open) | 2W | Am | T2 | 1 | 1.07 | 98.84 |
| | | | | | 2 | 1.08 | 98.84 |
| | | 1M | Am | T2 | 1 | 1.09 | 98.85 |
| | | | | | 2 | 1.09 | 98.84 |
| | | 2M | Am | T2 | 1 | 1.04 | 98.87 |
| | | | | | 2 | 1.04 | 98.87 |
| | | 3 M | Am | T2 | 1 | 1.01 | 98.87 |
| | | | | | 2 | 1.03 | 98.88 |

| | | | | | | | |
|---|---|---|---|---|---|---|---|
| *"RH": Relative humidity* *"W": week(s)* *"M": Month(s)* *"Am": amorphous;* *"T1": white powder;* *"T2": white or off white round tablet* | | | | | | | |

### Example 8: Tablets production

### Production of solid dispersions SD1 Powder and SD2 Powder (See Table 8 for formulation)

1. Methanol (for SD1 Powder) or a mixture of methanol and methylene chloride (1/1, v/v, for SD2 Powder) were transferred into a container and stirred using a mixer. While stirring, Compound (a) was added to the solvent. Stirring continued until Compound (a) dissolved. The resulting solution was filtered.
2. Eudragit L100 or hydroxypropyl methylcellulose E5 were added to the solution from step 1. The mixture was stirred until fully combined.
3. The two mixtures were spray dried using a suitable spray dryer (PSD-1) and the spray dried products were collected.

The resulting powders were dried using a tray oven dryer and collected.

**Table 9 Formulations for the preparation of the solid dispersions SD1 Powder and SD2 Powder**

| **Component** | **gram per 1000 g SD** | |
|---|---|---|
| | **SD1 Powder** | **SD2 Powder** |
| Compound (a) | 333.3 | 333.3 |
| methacrylic acid - methyl methacrylate copolymer (1: 1) | 666.7 | NA |
| Hydroxypropyl methylcellulose E5 | NA | 666.7 |
| Methanol" | 14384.6 | 7107.5 |
| Methylene Chloride^{a} | NA | 11890.6 |
| Total batch size | 1000 | 1000 |

| | | |
|---|---|---|
| *^{a} Removed (by drying) during processing* | | |

### Production of tablets comprising SD1 Powder (See Table 8 for formulation)

1. SD1 Powder, microcrystalline cellulose, mannitol, croscarmellose sodium, colloidal silicon dioxide, magnesium stearate, sodium lauryl sulfate, were sieved together and then blended in a bin blender.
2. The blend was roller compacted in a roller compactor WP-120 and the resulting granules were collected.
3. Croscarmellose sodium and microcrystalline cellulose were sieved together, and added to the dry granules obtained in step 2, the mixture was blended in a bin blender.
4. Add screened Magnesium stearate extra was added and the resulting mixture blended in a bin blender.
5. The final blend was compressed into tablets using a XL 100 Korsch tablet press.

**Table 10 Tablet formulation comprising SD1 Powder.**

| | **Ingredient** | **Tablet 7 50 mg API (mg/tablet)** | **Tablet 8 100 mg API (mg/tablet)** | **Tablet 9 200 mg API (mg/tablet)** |
|---|---|---|---|---|
| **Intra RC** | SD 1 Powder | 150 | 300 | 600 |
| | Mannitol | 24 | 48 | 96 |
| | Microcrystalline cellulose | 80 | 160 | 320 |
| | Sodium lauryl sulfate | 4.5 | 9 | 18 |
| | Croscarmellose Sodium | 12 | 24 | 48 |
| | Colloidal silicon dioxide | 6 | 12 | 24 |
| | Magnesium Stearate | 1.5 | 3 | 6 |
| **Extra RC** | Croscarmellose Sodium | 3 | 6 | 12 |
| | Microcrystalline cellulose | 17.5 | 35 | 70 |
| | Magnesium Stearate | 1.5 | 3 | 6 |
| **Total** | | 300 | 600 | 1200 |

### Production of tablets comprising SD2 Powder (See Table 9 for formulation)

1. SD2 Powder, microcrystalline cellulose, mannitol, croscarmellose sodium, colloidal silicon dioxide, magnesium stearate, sodium lauryl sulfate, were sieved together and then blended in a bin blender.
2. The blend was roller compacted in a roller compactor WP-120 and the resulting granules were collected.
3. Croscarmellose sodium and microcrystalline cellulose were sieved together, and added to the dry granules obtained in step 2, the mixture was blended in a bin blender.
4. Add screened Magnesium stearate extra was added and the resulting mixture blended in a bin blender.
5. The final blend was compressed into tablets using a XL 100 Korsch tablet press.

**Table 11 Tablet formulation comprising SD2 Powder.**

| | **Ingredient** | **Tablet 10 50 mg API (mg/tablet)** | **Tablet 11 100 mg API (mg/tablet)** | **Tablet 12 200 mg API (mg/tablet)** |
|---|---|---|---|---|
| **Intra RC** | SD2 Powder | 150 | 300 | 600 |
| | Mannitol | 24 | 48 | 96 |
| | Microcrystalline cellulose | 80 | 160 | 320 |
| | Sodium lauryl sulfate | 4.5 | 9 | 18 |
| | Croscarmellose Sodium | 12 | 24 | 48 |
| | Colloidal silicon dioxide | 6 | 12 | 24 |
| | Magnesium Stearate | 1.5 | 3 | 6 |
| **Extra RC** | Croscarmellose Sodium | 3 | 6 | 12 |
| | Microcrystalline cellulose | 17.5 | 35 | 70 |
| | Magnesium Stearate | 1.5 | 3 | 6 |
| **Total** | | 300 | 600 | 1200 |

**Table 12 Evaluation of produced tablets comprising SD1 Powder.**

| | **Tablet 7** | | | **Tablet 9** | | |
|---|---|---|---|---|---|---|
| **Main compression force (KN)** | 7.9 | 8.4-9.0 | 9.0-10.0 | 16.1-16.5 | 17.3-17.5 | 20.0-20.6 |

| **Hardness (n=5,N)** | | | | | | |
|---|---|---|---|---|---|---|
| **Min (N)** | 110 | 131 | 135 | 218 | 251 | 355 |
| **Max (N)** | 121 | 159 | 181 | 258 | 295 | 390 |
| **Average(N)** | 115.6 | 147.6 | 162.6 | 239.4 | 278 | 376.4 |

| **Friability (%):<0.5%** | | | | | | |
|---|---|---|---|---|---|---|
| | N/A | 0.0 | N/A | N/A | 0.0 | N/A |

| **Disintegration time (n** = **6, mm:ss): <15 min** | | | | | | |
|---|---|---|---|---|---|---|
| **First** | 00:45 | 02:04 | 03:23 | 01:16 | 02:11 | 04:40 |
| **Last** | 01:22 | 02:46 | 05:24 | 01:38 | 02:57 | 05:10 |

**Table 13 Evaluation of produced tablets comprising SD2 Powder.**

| | **Tablet 10** | | | **Tablet 12** | | |
|---|---|---|---|---|---|---|
| **Main compression force (KN)** | 7.7-8.4 | 8.4-9.2 | 10.1 | 15.6-16.3 | 15.7-17.0 | 17.4-18.0 |

| **Hardness (n=5,N)** | | | | | | |
|---|---|---|---|---|---|---|
| **Min (N)** | 107 | 13₄ | 158 | 230 | 268 | 282 |
| **Max (N)** | 124 | 166 | 191 | 259 | 305 | 322 |
| **Average(N)** | 115.4 | 146.8 | 175.8 | 242.3 | 288.7 | 297.3 |

| **Friability (%):<0.5%** | | | | | | |
|---|---|---|---|---|---|---|
| | 0.0 | N/A | N/A | N/A | 0.0 | N/A |

| **Disintegration time (n** = **6, mm:ss): <15 min** | | | | | | |
|---|---|---|---|---|---|---|
| **First** | 02:14 | 03:27 | 05:35 | 02:38 | 04:43 | 04:20 |
| **Last** | 03:40 | 03:50 | 07:24 | 03:53 | 04:45 | 05:25 |

### Example 9: pharmacokinetics after oral administration of the tablets in fasted dogs.

A pharmacokinetic (PK) study was carried out using tablets 7 and 10 mentioned above. The tablets were orally administered once, twice or 3 times to male beagle dogs (N=3) in a fasted condition. Results are shown in Table 14.

**Table 14. Fasted dog pharmacokinetics at different doses**

| | **Tablet 7** | | **Tablet 10** | | |
|---|---|---|---|---|---|
| **Dose** | 50 mg/dog | 2*50 mg/dog | 50 mg/dog | 2*50 mg/dog | 3*50 mg/dog |
| **N** | 3 | 3 | 3 | 3 | 3 |
| **Cₘₐₓ (ng/mL)** | 1320±499 | 1680±327 | 873±123 | 2160±385 | 2590 ± 996 |
| **Tₘₐₓ(h)** | 4.00 (4.00-4.00) | 4.00 (4.00-7.00) | 4.00 (4.00-4.00) | 4.00 (4.00-4.00) | 4.00 (4.00-4.00) |
| **AUCₗₐₛₜ (ng·h/mL** ) | 22600±4300 | 30000±1230 0 | 13500±2810 | 22300±8210 | 42800 ± 21000 |
| **AUC_{∞} (ng·h/mL** ) | 23500±4550 | 30800±1310 0 | 13800±2860 | 22700±8610 | 43100 ± 21200 |

| | | | | | |
|---|---|---|---|---|---|
| *Mean + standard deviation* *A UC = area under the plasma concentration-time curve; AUCₗₐₛₜ = A UC calculated until the last timepoint of blood plasma level; AUC_{∞} = AUC calculated using extrapolation of the plasma profile to infinity time;* *Cₘₐₓ = maximum observed plasma concentration; N = number of animals; Tₘₐₓ = time correspondent to the maximum observed plasma concentration.* | | | | | |

### Example 10: pharmacokinetics after oral administration of the tablets in fasted and fed dogs.

A pharmacokinetic (PK) study was carried out using tablets 9 and 12 mentioned above. Three tablets were orally administered to male beagle dogs (N=3) in a fasted and fed conditions. Results are shown in Table 15.

**Table 15. Fasted and fed dog pharmacokinetics**

| | **Tablet 9** | | **Tablet 12** | |
|---|---|---|---|---|
| **Food** | **Fasted** | **Fed** | **Fasted** | **Fed** |
| **Dose** | 200 mg *3 | 200 mg *3 | 200 mg *3 | 200 mg *3 |
| **N** | 3 | 3 | 3 | 3 |
| **Cₘₐₓ (ng/mL)** | 6410 ± 874 | 8540 ± 1160 | 7380 ± 4460 | 12300 ± 1790 |
| **Tₘₐₓ (h)** | 7.00 (7.00 - 7.00) | 7.00 (4.00 - 7.00) | 7.00 (4.00 - 24.00) | 7.00 (7.00 - 7.00) |
| **AUCₗₐₛₜ (ng·h/mL)** | 139000 ± 26700 | 328000 ± 136000 | 222000 ± 156000 | 385000 ± 13500 |
| **AUC_{∞} (ng·h/mL)** | 140000 ± 27100 | 330000 ± 138000 | 223000 ± 157000 | 387000 ± 13900 |

| | | | | |
|---|---|---|---|---|
| *Mean* ± *standard deviation* *AUC* = *area under the plasma concentration-time curve; AUCₗₐₛₜ = AUC calculated until the last timepoint of blood plasma level; AUC_{∞} = AUC calculated using extrapolation of the plasma profile to infinity time;* *Cₘₐₓ = maximum observed plasma concentration; N = number of animals; Tₘₐₓ = time correspondent to the maximum observed plasma concentration.* | | | | |

The pharmacokinetic data in dogs clearly indicated that the AUC values were similar for fasted and fed state, indicating the absence of a food effect. Hence, this formulation releases the API independent of the presence or absence of the food. This is beneficial from a patient compliance point of view. Therefore, a formulation according to the invention may result in a reduced food effect compared to other formulations.

While preferred embodiments of the present invention have been shown and described herein, it will be apparent to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention and that embodiments within the scope of these claims and their equivalents be covered thereby.

## Claims

1. A solid dosage form comprising a pharmaceutical formulation, comprising
a) an active pharmaceutical ingredient (API); and
b1) methacrylic acid copolymer, or
b2) a cellulose derivative;
wherein the active pharmaceutical ingredient is a dengue viral replication inhibitor of formula : 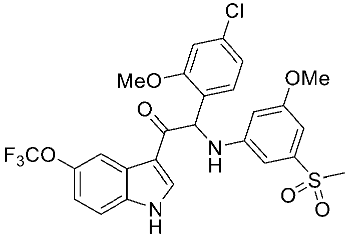 or a stereo-isomeric form, a pharmaceutically acceptable salt, solvate or polymorph thereof.

2. The solid dosage form of claim 1, wherein the viral replication inhibitor is the (S)-enantiomer of

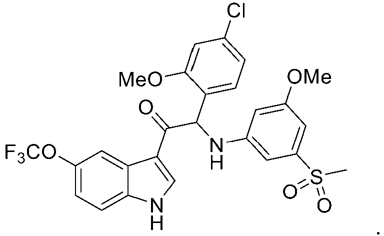

3. The solid dosage form of any one of claims 1 or 2, wherein the active pharmaceutical ingredient and one of the methacrylic acid copolymer or the cellulose derivative are present in said formulation in a ratio of from 4:1 w/w to 1:5 w/w.

4. The solid dosage form of any one of claims 1 to 3, wherein the methacrylic acid copolymer is selected from the group comprising a copolymer of methacrylic acid and methyl methacrylate; a copolymer of methacrylic acid and ethyl acrylate and mixture thereof.

5. The solid dosage form of any one of claims 1 to 3, wherein the cellulose derivative has a viscosity ranging between 3 and 5000 mPa.s in 2 wt% solution in H2O at 25°C and is selected from the group comprising HPMC E5, HPMC E6, HPMC E15, HPMC E50, HPMC K4M and HPMC-AS.

6. The solid dosage form of any one of claims 1 to 5, wherein the pharmaceutical formulation comprises at most 40 wt%, preferably at most 30 wt%, preferably at most 25 wt% of the active pharmaceutical ingredient relative to the total weight of the formulation.

7. The solid dosage form of any one of claims 1 to 6, wherein the pharmaceutical formulation further comprises one or more pharmaceutically acceptable excipients selected from disintegrants, binders, diluents, lubricants, stabilizers, wetting agents, glidants, osmotic agents, colorants, plasticizers, and coatings.

8. The solid dosage form of any one of claims 1 to 7, wherein the formulation comprises a plurality of granules forming an intragranular phase of the formulation and one or more excipients forming an extragranular phase of the formulation.

9. The solid dosage form of any one of claims 1-8, wherein the dosage form is an oral dosage form.

10. The solid dosage form of any one of claims 1-9, wherein the dosage form is a tablet.

11. The solid dosage form of any one of claims 1-10, wherein the formulation comprises from 0.5 mg to 1000 mg of the active pharmaceutical ingredient; preferably the formulation comprises from 1 mg to 1000 mg of the active pharmaceutical ingredient; preferably the formulation comprises from 2 mg to 500 mg of the active pharmaceutical ingredient.

12. The solid dosage form of any one of claims 1-11, wherein the formulation comprises at least 0.5 mg of the active pharmaceutical ingredient, preferably at least 5 mg, preferably at least 10 mg, preferably at least 20 mg, preferably at least 50 mg, preferably at least 100 mg, preferably at least 200 mg, preferably at least 300 mg, preferably at least 400 mg, preferably at least 500 mg or preferably at least 1000 mg of the active pharmaceutical ingredient.

13. A solid dosage form of any one of claims 1 to 12 for use in treating or preventing dengue viral infections.

14. A process for preparing a solid dosage form according to any one of claims 1 to 13, comprising the steps of:
a) dissolving the API in a solvent to form a solution;
b) mixing the methacrylic acid copolymer or the cellulose derivative or a combination thereof with the solution formed in (a) thereby obtaining a mixture;
c) spray drying the mixture to obtain a solid dispersion;
d) optionally blending the solid dispersion with at least one pharmaceutically acceptable excipient;
e) compressing the blend into a tablet;
to provide a solid dosage form according to any one of claims 1 to 13.

15. The process of claim 14, wherein the active pharmaceutical ingredient is a dengue viral replication inhibitor of formula : 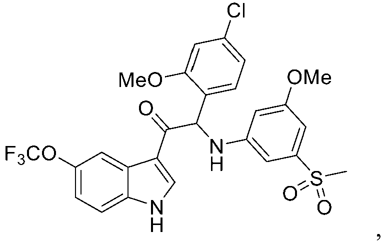 preferably the (S)-enantiomer,
or a stereo-isomeric form, a pharmaceutically acceptable salt, solvate or polymorph thereof.
